# EUROPEAN PATENT SPECIFICATION

(11) **EP 2 750 707 B1**
(45) Date of publication and mention of the grant of the patent: **24.10.2018**
(21) Application number: 12769787.8
(22) Date of filing: 31.08.2012
(51) Int. Cl.: A61K 39/39, A61K 9/127, A61K 9/00

(54) **PEGYLATED LIPOSOMES FOR DELIVERY OF IMMUNOGEN-ENCODING RNA**
PEGYLIERTE LIPOSOME ZUR FREISETZUNG VON IMMUNOGEN-CODIERENDER RNA
LIPOSOMES PÉGYLÉS POUR L'ADMINISTRATION D'ARN CODANT UN IMMUNOGÈNE

(30) Priority: 31.08.2011 US 201161529878 P
(43) Date of publication of application: 09.07.2014
(73) Proprietor: GlaxoSmithKline Biologicals SA, 1330 Rixensart (BE)
(72) Inventor: GEALL, Andrew, Emeryville, CA 94608-2916 (US); VERMA, Ayush, Emeryville, CA 94608-2916 (US)
(74) Representative: Sanderson, Andrew John
(86) International application number: PCT/US2012/053391
(87) International publication number: WO 2013/033563

(56) References cited:
- EP-A1- 1 083 232
- WO-A1-2012/030901
- WO-A2-2010/088537
- WO-A2-2011/008974
- WO-A2-2012/006376
- MARYAM AMIDI ET AL: "Antigen-expressing immunostimulatory liposomes as a genetically programmable synthetic vaccine", SYSTEMS AND SYNTHETIC BIOLOGY, SPRINGER NETHERLANDS, DORDRECHT, vol. 5, no. 1 - 2, 26 October 2010 (2010-10-26), pages 21-31, XP019943076, ISSN: 1872-5333, DOI: 10.1007/S11693-010-9066-Z
- HIROSHI KITA ET AL: "Replication of Genetic Information with Self-Encoded Replicase in Liposomes", CHEMBIOCHEM, vol. 9, no. 15, 13 October 2008 (2008-10-13), pages 2403-2410, XP055007329, ISSN: 1439-4227, DOI: 10.1002/cbic.200800360
- KLIBANOV A L ET AL: "Amphipathic polyethyleneglycols effectively prolong the circulation time of liposomes", FEBS LETTERS, ELSEVIER, AMSTERDAM, NL, vol. 268, no. 1, 30 July 1990 (1990-07-30) , pages 235-237, XP025890437, ISSN: 0014-5793, DOI: 10.1016/0014-5793(90)81016-H [retrieved on 1990-07-30]
- ABDERRAHIM AISSAOUI ET AL: "Efficient topical delivery of plasmid DNA to lung in vivo mediated by putative triggered, PEGylated pDNA nanoparticles", JOURNAL OF CONTROLLED RELEASE, ELSEVIER, AMSTERDAM, NL, vol. 154, no. 3, 4 June 2011 (2011-06-04), pages 275-284, XP028285878, ISSN: 0168-3659, DOI: 10.1016/J.JCONREL.2011.06.017 [retrieved on 2011-06-15]
- MARYAM AMIDI ET AL: "Induction of humoral and cellular immune responses by antigen-expressing immunostimulatory liposomes", JOURNAL OF CONTROLLED RELEASE, 1 August 2012 (2012-08-01), XP055044026, ISSN: 0168-3659, DOI: 10.1016/j.jconrel.2012.08.016

## Description

### TECHNICAL FIELD

This invention is in the field of non-viral delivery of RNA for immunisation.

### BACKGROUND ART

The delivery of nucleic acids for immunising animals has been a goal for several years. Various approaches have been tested, including the use of DNA or RNA, of viral or non-viral delivery vehicles (or even no delivery vehicle, in a "naked" vaccine), of replicating or non-replicating vectors, or of viral or non-viral vectors. Amidi et al., Syst.Synth..Biol. (2011) 5:21-31discloses antigen expressing immunostimulatory liposomes which are made from DSPE-PEG5000. WO 2011/08974 and WO 2010/088537 both disclose liposomes comprising RNA in which the liposomes contain PEG2000.

There remains a need for further and improved nucleic acid vaccines and, in particular, for improved ways of delivering nucleic acid vaccines.

### DISCLOSURE OF THE INVENTION

The scope of the invention is defined by the claims. According to the invention, nucleic acid immunisation is achieved by delivering RNA encapsulated within a liposome. The RNA encodes an immunogen of interest. The liposome includes a PEGylated lipid *i.e.* the lipid is modified by covalent attachment of a polyethylene glycol. PEG provides the liposomes with a coat which can confer favourable pharmacokinetic characteristics *e.g.* it can increase stability and prevent non-specific adsorption of the liposomes. The inventors have found that the length of the PEG can affect *in vivo* expression of encapsulated RNA and so the invention uses liposomes which comprise PEG which has an average molecular mass above 3kDa but less than 11kDa. PEG with a molecular weight below 1kDa (*e.g.* 500 or 750 Da) does not form stable liposomes, and liposomes formed with PEG in the range of 1-3kDa have shown lower efficacy in immunogenicity experiments (see below).

Thus the invention provides a liposome within which RNA encoding an immunogen of interest is encapsulated, wherein the liposome comprises at least one lipid which includes a polyethylene glycol moiety, such that polyethylene glycol is present on the liposome's exterior, wherein the average molecular mass of the polyethylene glycol is above 3kDa but less than 11kDa. These liposomes are suitable for *in vivo* delivery of the RNA to a vertebrate cell and so they are useful as components in pharmaceutical compositions for immunising subjects against various diseases.

Disclosed is a process for preparing a RNA-containing liposome, comprising a step of mixing RNA with one or more lipids, under conditions such that the lipids form a liposome in which the RNA is encapsulated, wherein at least one lipid includes a polyethylene glycol moiety which becomes located on the liposome's exterior during the process, and wherein the average molecular mass of the polyethylene glycol is above 3kDa but less than 11kDa.

### The liposome

The invention utilises liposomes within which immunogen-encoding RNA is encapsulated. Thus the RNA is (as in a natural virus) separated from any external medium. Encapsulation within the liposome has been found to protect RNA from RNase digestion. The liposomes can include some external RNA (*e.g.* on their surface), but at least half of the RNA (and ideally all of it) is encapsulated in the liposome's core. Encapsulation within liposomes is distinct from, for instance, the lipid/RNA complexes disclosed in reference 1, where RNA is mixed with pre-formed liposomes.

Various amphiphilic lipids can form bilayers in an aqueous environment to encapsulate a RNA-containing aqueous core as a liposome. These lipids can have an anionic, cationic or zwitterionic hydrophilic head group. Formation of liposomes from anionic phospholipids dates back to the 1960s, and cationic liposome-forming lipids have been studied since the 1990s. Some phospholipids are anionic whereas other are zwitterionic and others are cationic. Suitable classes of phospholipid include, but are not limited to, phosphatidylethanolamines, phosphatidylcholines, phosphatidylserines, and phosphatidyl-glycerols, and some useful phospholipids are listed in Table 1.

Useful cationic lipids include, but are not limited to, dioleoyl trimethylammonium propane (DOTAP), 1,2-distearyloxy-N,N-dimethyl-3-aminopropane (DSDMA), 1,2-dioleyloxy-N,Ndimethyl-3-aminopropane (DODMA), 1,2-dilinoleyloxy-N,N-dimethyl-3-aminopropane (DLinDMA), 1,2-dilinolenyloxy-N,N-dimethyl-3-aminopropane (DLenDMA); further useful cationic lipids are disclosed in references 2 and 3. Zwitterionic lipids include, but are not limited to, acyl zwitterionic lipids and ether zwitterionic lipids. Examples of useful zwitterionic lipids are DPPC, DSPC, DOPC, dodecylphosphocholine, 1,2-dioleoyl-sn-glycero-3-phosphatidylethanolamine (DOPE), and 1,2-diphytanoyl-sn-glycero-3-phosphoethanolamine (DPyPE). The lipids can be saturated or unsaturated. The use of at least one unsaturated lipid for preparing liposomes is preferred. If an unsaturated lipid has two tails, both tails can be unsaturated, or it can have one saturated tail and one unsaturated tail. A lipid can include a steroid group in one tail *e.g.* as in RV05.

Thus in one embodiment the invention provides a liposome having a lipid bilayer encapsulating an aqueous core, wherein: (i) the lipid bilayer comprises at least one lipid which includes a polyethylene glycol moiety, such that polyethylene glycol is present on the liposome's exterior, wherein the average molecular mass of the polyethylene glycol is above 3kDa but less than 11kDa; and (ii) the aqueous core includes a RNA which encodes an immunogen.

Liposomes can be formed from a single lipid or from a mixture of lipids. A mixture may comprise (i) a mixture of anionic lipids (ii) a mixture of cationic lipids (iii) a mixture of zwitterionic lipids (iv) a mixture of anionic lipids and cationic lipids (v) a mixture of anionic lipids and zwitterionic lipids (vi) a mixture of zwitterionic lipids and cationic lipids or (vii) a mixture of anionic lipids, cationic lipids and zwitterionic lipids. Similarly, a mixture may comprise both saturated and unsaturated lipids. For example, a mixture may comprise DSPC (zwitterionic, saturated), DlinDMA (cationic, unsaturated), and/or DMG (anionic, saturated). Where a mixture of lipids is used, not all of the component lipids in the mixture need to be amphiphilic e.g. one or more amphiphilic lipids can be mixed with cholesterol.

Where a liposome of the invention is formed from a mixture of lipids, it is preferred that the proportion of those lipids which are PEGylated as described herein is less than 10% of the total amount of lipids *e.g*. between 0.5-5%, between 1-4%, or about 2%. For instance, useful liposomes are shown below in which 2% of the total lipid is a PEG-DMG. The remainder can be made of *e.g.* cholesterol (*e.g.* 35-50% cholesterol) and/or cationic lipid (*e.g.* 30-70%) and/or DSPC (*e.g.* 5-15%). Such mixtures are used below. These percentage values are mole percentages.

Thus a liposome can be formed from a cationic lipid (*e.g.* DlinDMA, RV05), a zwitterionic lipid (*e.g.* DSPC, DPyPE), a cholesterol, and a PEGylated lipid. A mixture of DSPC, DlinDMA, PEG-DMG and cholesterol is used in the examples, as well as several further mixtures.

At least one lipid within the liposome includes a polyethylene glycol moiety. Liposomes which include these PEGylated lipids will have PEG oriented so that it is present on at least the exterior of the liposome (but some PEG may also be exposed to the liposome's interior *i.e.* to the aqueous core). This orientation can be achieved by attaching the PEG to an appropriate part of the lipid. For instance, in an amphiphilic lipid the PEG would be attached to the hydrophilic head, as it is this head which orients itself to the lipid bilayer's aqueous-facing exterior. PEGylation in this way can be achieved by covalent attachment of a PEG to a lipid *e.g.* using techniques such as those disclosed in reference 1 and 2.

Thus the PEGylated lipids will comprise the PEG structure: 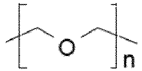 where n provides a molecular weight for the PEG of above 3kDa but less than 11kDa *e.g.* 69 or more, or between 70 and 240, or about 113 for a 5kDa PEGylation.

The PEG moiety can terminate with an -O-methyl group, and so a PEGylated lipid may comprise:

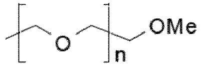

Including attachment to a nitrogen in a lipid's head group, therefore, a PEGylated lipid useful with the invention may comprise:

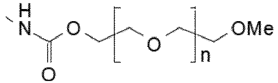

One suitable PEGylated lipid for use with the invention is PEG-DMG, as used in the examples. Other PEGylated lipids can be used *e.g.* lipids of Formula (X): 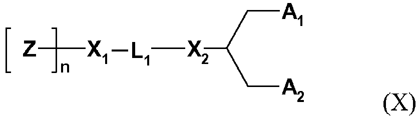 wherein:
[Z]ₙ is a hydrophilic PEG head group component wherein the polymer may be linear or branched, and wherein the polymer may be optionally substituted;
Z is polymerized by n subunits;
n is a number-averaged degree of polymerization between 10 and 200 units of Z (and can be optimized for different Z groups);
L₁ is an optionally substituted C₁₋₁₀ alkylene or C₁₋₁₀ heteroalkylene linker including zero, one or two of an ether (e.g., -O-), ester (e.g., -C(O)O-), succinate (e.g., -O(O)C-CH₂-CH₂-C(O)O-)), carbamate (e.g., -OC(O)-NR'-), carbonate (e.g., -OC(O)O-), urea (e.g., -NRC(O)NR'-), amine (e.g., -NR'-), amide (e.g., -C(O)NR'-), imine (e.g., -C(NR')-), thioether (e.g., -S-), xanthate (e.g., -OC(S)S-), and phosphodiester (e.g., -OP(O)₂O-), wherein R' is independently selected from -H, -NH-, -NH₂, -O-, -S-, a phosphate or an optionally substituted C₁₋₁₀ alkylene;
X₁ and X₂ are independently selected from a carbon or a heteroatom selected from - NH-, -O-, -S- or a phosphate;
A₁ and A₂ are either independently selected from a C₆₋₃₀ alkyl, C₆₋₃₀ alkenyl, and C₆₋₃₀ alkynyl, wherein A₁ and A₂ may be the same or different, or A₁ and A₂ together with the carbon atom to which they are attached form an optionally substituted steroid.

A liposome of the invention will typically include a large number of PEG moieties, which may be the same or different. The average molecular mass of the PEG in a liposome of the invention is above 3kDa but less than 11kDa *e.g*. between 3.5-9kDa, between 4-7.5kDa, between 4.5-6kDa, between 4.8-5.5kDa, or 5kDa. Thus the PEG can be a PEG which is commonly known as "PEG 5000" or "PEG 5k". In some embodiments the invention does not encompass liposomes which comprise a PEG-conjugated lipid in which the PEG has an average molecular mass of 8kDa; in some embodiments the invention does not encompass liposomes which comprise a PEG-conjugated lipid in which the PEG has an average molecular mass of between 7.9-8.1kDa.

The PEG will usually comprise linear polymer chains but, in some embodiments, the PEG may comprise branched polymer chains.

In some embodiments the PEG may be a substituted PEG *e.g.* in which one or more carbon atoms in the polymer is substituted by one or more alkyl, alkoxy, acyl or aryl groups.

In some embodiments the PEG may include copolymer groups *e.g.* one or more propylene monomers, to form a PEG polypropylene polymer.

Liposomes are usually divided into three groups: multilamellar vesicles (MLV); small unilamellar vesicles (SUV); and large unilamellar vesicles (LUV). MLVs have multiple bilayers in each vesicle, forming several separate aqueous compartments. SUVs and LUVs have a single bilayer encapsulating an aqueous core; SUVs typically have a diameter ≤50nm, and LUVs have a diameter >50nm. Liposomes of the invention are ideally LUVs with a diameter in the range of 60-180nm, and preferably in the range of 80-160nm.

A liposome of the invention can be part of a composition comprising a plurality of liposomes, and the liposomes within the plurality can have a range of diameters. For a composition comprising a population of liposomes with different diameters: (i) at least 80% by number of the liposomes should have diameters in the range of 60-180nm, and preferably in the range of 80-160nm, and/or (ii) the average diameter (by intensity *e.g.* Z-average) of the population is ideally in the range of 60-180nm, and preferably in the range of 80-160nm. The diameters within the plurality should ideally have a polydispersity index <0.2. The liposome/RNA complexes of reference 1 are expected to have a diameter in the range of 600-800nm and to have a high polydispersity.

Techniques for preparing suitable liposomes are well known in the art *e.g.* see references 6 to 8. One useful method is described in reference 9 and involves mixing (i) an ethanolic solution of the lipids (ii) an aqueous solution of the nucleic acid and (iii) buffer, followed by mixing, equilibration, dilution and purification. Preferred liposomes of the invention are obtainable by this mixing process. To obtain liposomes with the desired diameter(s), mixing can be performed using a process in which two feed streams of aqueous RNA solution are combined in a single mixing zone with one stream of an ethanolic lipid solution, all at the same flow rate *e.g.* in a microfluidic channel as described below.

### The RNA

Liposomes of the invention include a RNA molecule which (unlike siRNA, as in reference 4) encodes an immunogen. After *in vivo* administration of the particles, RNA is released from the particles and is translated inside a cell to provide the immunogen *in situ.*

The RNA is +-stranded, and so it can be translated by cells without needing any intervening replication steps such as reverse transcription. It can also bind to TLR7 receptors expressed by immune cells, thereby initiating an adjuvant effect.

Preferred +-stranded RNAs are self-replicating. A self-replicating RNA molecule (replicon) can, when delivered to a vertebrate cell even without any proteins, lead to the production of multiple daughter RNAs by transcription from itself (via an antisense copy which it generates from itself). A self-replicating RNA molecule is thus typically a +-strand molecule which can be directly translated after delivery to a cell, and this translation provides a RNA-dependent RNA polymerase which then produces both antisense and sense transcripts from the delivered RNA. Thus the delivered RNA leads to the production of multiple daughter RNAs. These daughter RNAs, as well as collinear subgenomic transcripts, may be translated themselves to provide *in situ* expression of an encoded immunogen, or may be transcribed to provide further transcripts with the same sense as the delivered RNA which are translated to provide *in situ* expression of the immunogen. The overall results of this sequence of transcriptions is a huge amplification in the number of the introduced replicon RNAs and so the encoded immunogen becomes a major polypeptide product of the cells.

One suitable system for achieving self-replication is to use an alphavirus-based RNA replicon. These +-stranded replicons are translated after delivery to a cell to give of a replicase (or replicase-transcriptase). The replicase is translated as a polyprotein which auto-cleaves to provide a replication complex which creates genomic --strand copies of the +-strand delivered RNA. These --strand transcripts can themselves be transcribed to give further copies of the +-stranded parent RNA and also to give a subgenomic transcript which encodes the immunogen. Translation of the subgenomic transcript thus leads to *in situ* expression of the immunogen by the infected cell. Suitable alphavirus replicons can use a replicase from a sindbis virus, a semliki forest virus, an eastern equine encephalitis virus, a venezuelan equine encephalitis virus, *etc.* Mutant or wild-type viruses sequences can be used *e.g.* the attenuated TC83 mutant of VEEV has been used in replicons [10].

A preferred self-replicating RNA molecule thus encodes (i) a RNA-dependent RNA polymerase which can transcribe RNA from the self-replicating RNA molecule and (ii) an immunogen. The polymerase can be an alphavirus replicase e.g. comprising one or more of alphavirus proteins nsP1, nsP2, nsP3 and nsP4.

Whereas natural alphavirus genomes encode structural virion proteins in addition to the non-structural replicase polyprotein, it is preferred that a self-replicating RNA molecule of the invention does not encode alphavirus structural proteins. Thus a preferred self-replicating RNA can lead to the production of genomic RNA copies of itself in a cell, but not to the production of RNA-containing virions. The inability to produce these virions means that, unlike a wild-type alphavirus, the self-replicating RNA molecule cannot perpetuate itself in infectious form. The alphavirus structural proteins which are necessary for perpetuation in wild-type viruses are absent from self-replicating RNAs of the invention and their place is taken by gene(s) encoding the immunogen of interest, such that the subgenomic transcript encodes the immunogen rather than the structural alphavirus virion proteins.

Thus a self-replicating RNA molecule useful with the invention may have two open reading frames. The first (5') open reading frame encodes a replicase; the second (3') open reading frame encodes an immunogen. In some embodiments the RNA may have additional (*e.g*. downstream) open reading frames e.g. to encode further immunogens (see below) or to encode accessory polypeptides.

A self-replicating RNA molecule can have a 5' sequence which is compatible with the encoded replicase.

Self-replicating RNA molecules can have various lengths but they are typically 5000-25000 nucleotides long *e.g.* 8000-15000 nucleotides, or 9000-12000 nucleotides. Thus the RNA is longer than seen in siRNA delivery.

A RNA molecule useful with the invention may have a 5' cap (*e.g.* a 7-methylguanosine). This cap can enhance *in vivo* translation of the RNA.

The 5' nucleotide of a RNA molecule useful with the invention may have a 5' triphosphate group. In a capped RNA this may be linked to a 7-methylguanosine via a 5'-to-5' bridge. A 5' triphosphate can enhance RIG-I binding and thus promote adjuvant effects.

A RNA molecule may have a 3' poly-A tail. It may also include a poly-A polymerase recognition sequence (*e.g*. AAUAAA) near its 3' end.

A RNA molecule useful with the invention will typically be single-stranded. Single-stranded RNAs can generally initiate an adjuvant effect by binding to TLR7, TLR8, RNA helicases and/or PKR. RNA delivered in double-stranded form (dsRNA) can bind to TLR3, and this receptor can also be triggered by dsRNA which is formed either during replication of a single-stranded RNA or within the secondary structure of a single-stranded RNA.

A RNA molecule useful with the invention can conveniently be prepared by *in vitro* transcription (IVT). IVT can use a (cDNA) template created and propagated in plasmid form in bacteria, or created synthetically (for example by gene synthesis and/or polymerase chain-reaction (PCR) engineering methods). For instance, a DNA-dependent RNA polymerase (such as the bacteriophage T7, T3 or SP6 RNA polymerases) can be used to transcribe the RNA from a DNA template. Appropriate capping and poly-A addition reactions can be used as required (although the replicon's poly-A is usually encoded within the DNA template). These RNA polymerases can have stringent requirements for the transcribed 5' nucleotide(s) and in some embodiments these requirements must be matched with the requirements of the encoded replicase, to ensure that the IVT-transcribed RNA can function efficiently as a substrate for its self-encoded replicase.

As discussed in reference 11, the self-replicating RNA can include (in addition to any 5' cap structure) one or more nucleotides having a modified nucleobase. For instance, a self-replicating RNA can include one or more modified pyrimidine nucleobases, such as pseudouridine and/or 5-methylcytosine residues. In some embodiments, however, the RNA includes no modified nucleobases, and may include no modified nucleotides *i.e.* all of the nucleotides in the RNA are standard A, C, G and U ribonucleotides (except for any 5' cap structure, which may include a 7'-methylguanosine). In other embodiments, the RNA may include a 5' cap comprising a 7'-methylguanosine, and the first 1, 2 or 3 5' ribonucleotides may be methylated at the 2' position of the ribose.

A RNA used with the invention ideally includes only phosphodiester linkages between nucleosides, but in some embodiments it can contain phosphoramidate, phosphorothioate, and/or methylphosphonate linkages.

Ideally, a liposome includes fewer than 10 different species of RNA *e.g.* 5, 4, 3, or 2 different species; most preferably, a liposome includes a single RNA species *i.e.* all RNA molecules in the liposome have the same sequence and same length.

The amount of RNA per liposome can vary. The number of individual self-replicating RNA molecules per liposome is typically ≤50 *e.g.* <20, <10, <5, or 1-4 per liposome.

### The immunogen

RNA molecules used with the invention encode a polypeptide immunogen. After administration of the liposomes the RNA is translated *in vivo* and the immunogen can elicit an immune response in the recipient. The immunogen may elicit an immune response against a bacterium, a virus, a fungus or a parasite (or, in some embodiments, against an allergen; and in other embodiments, against a tumor antigen). The immune response may comprise an antibody response (usually including IgG) and/or a cell-mediated immune response. The polypeptide immunogen will typically elicit an immune response which recognises the corresponding bacterial, viral, fungal or parasite (or allergen or tumour) polypeptide, but in some embodiments the polypeptide may act as a mimotope to elicit an immune response which recognises a bacterial, viral, fungal or parasite saccharide. The immunogen will typically be a surface polypeptide *e.g.* an adhesin, a hemagglutinin, an envelope glycoprotein, a spike glycoprotein, *etc.*

The RNA molecule can encode a single polypeptide immunogen or multiple polypeptides. Multiple immunogens can be presented as a single polypeptide immunogen (fusion polypeptide) or as separate polypeptides. If immunogens are expressed as separate polypeptides from a replicon then one or more of these may be provided with an upstream IRES or an additional viral promoter element. Alternatively, multiple immunogens may be expressed from a polyprotein that encodes individual immunogens fused to a short autocatalytic protease (*e.g*. foot-and-mouth disease virus 2A protein), or as inteins.

Unlike references 1 and 12, the RNA encodes an immunogen. For the avoidance of doubt, the invention does not encompass RNA which encodes a firefly luciferase or which encodes a fusion protein of *E.coli* β-galactosidase or which encodes a green fluorescent protein (GFP). Such polypeptides may be useful as markers, or even in a gene therapy context, but the invention concerns delivery of RNA for eliciting an immunological response system. Thus the immunogen also is not a self protein which is delivered to supplement or substitute for a defective host protein (as in gene therapy). Also, the RNA is not total mouse thymus RNA.

In some embodiments the immunogen elicits an immune response against one of these bacteria:
*Neisseria meningitidis:* useful immunogens include, but are not limited to, membrane proteins such as adhesins, autotransporters, toxins, iron acquisition proteins, and factor H binding protein. A combination of three useful polypeptides is disclosed in reference 13.
*Streptococcus pneumoniae:* useful polypeptide immunogens are disclosed in reference 14. These include, but are not limited to, the RrgB pilus subunit, the beta-N-acetyl-hexosaminidase precursor (spr0057), spr0096, General stress protein GSP-781 (spr2021, SP2216), serine/threonine kinase StkP (SP1732), and pneumococcal surface adhesin PsaA.
*Streptococcus pyogenes:* useful immunogens include, but are not limited to, the polypeptides disclosed in references 15 and 16.
*Moraxella catarrhalis.*
*Bordetella pertussis:* Useful pertussis immunogens include, but are not limited to, pertussis toxin or toxoid (PT), filamentous haemagglutinin (FHA), pertactin, and agglutinogens 2 and 3.
*Staphylococcus aureus:* Useful immunogens include, but are not limited to, the polypeptides disclosed in reference 17, such as a hemolysin, esxA, esxB, ferrichrome-binding protein (sta006) and/or the sta011 lipoprotein.
*Clostridium tetani:* the typical immunogen is tetanus toxoid.
*Cornynebacterium diphtheriae:* the typical immunogen is diphtheria toxoid.
*Haemophilus influenzae:* Useful immunogens include, but are not limited to, the polypeptides disclosed in references 18 and 19.
*Pseudomonas aeruginosa*
*Streptococcus agalactiae:* useful immunogens include, but are not limited to, the polypeptides disclosed in reference 15.
*Chlamydia trachomatis:* Useful immunogens include, but are not limited to, PepA, LcrE, ArtJ, DnaK, CT398, OmpH-like, L7/L12, OmcA, AtoS, CT547, Eno, HtrA and MurG (*e.g*. as disclosed in reference 20. LcrE [21] and HtrA [22] are two preferred immunogens.
*Chlamydia pneumoniae:* Useful immunogens include, but are not limited to, the polypeptides disclosed in reference 23.
*Helicobacter pylori:* Useful immunogens include, but are not limited to, CagA, VacA, NAP, and/or urease [24].
*Escherichia coli:* Useful immunogens include, but are not limited to, immunogens derived from enterotoxigenic *E. coli* (ETEC), enteroaggregative *E. coli* (EAggEC), diffusely adhering *E*. *coli* (DAEC), enteropathogenic *E. coli* (EPEC), extraintestinal pathogenic *E. coli* (ExPEC) and/or enterohemorrhagic *E. coli* (EHEC). ExPEC strains include uropathogenic *E.coli* (UPEC) and meningitis/sepsis-associated *E.coli* (MNEC). Useful UPEC polypeptide immunogens are disclosed in references 25 and 26. Useful MNEC immunogens are disclosed in reference 27. A useful immunogen for several *E.coli* types is AcfD [28].
*Bacillus anthracis*
*Yersinia pestis:* Useful immunogens include, but are not limited to, those disclosed in references 29 and 30.
*Staphylococcus epidermis*
*Clostridium perfringens* or *Clostridium botulinums*
*Legionella pneumophila*
*Coxiella burnetii*
*Brucella,* such as *B.abortus, B.canis, B.melitensis, B.neotomae, B.ovis, B.suis, B.pinnipediae.*
*Francisella,* such as *F.novicida, F.philomiragia, F.tularensis.*
*Neisseria gonorrhoeae*
*Treponema pallidum*
*Haemophilus ducreyi*
*Enterococcus faecalis* or *Enterococcus faecium*
*Staphylococcus saprophyticus*
*Yersinia enterocolitica*
*Mycobacterium tuberculosis*
*Rickettsia*
*Listeria monocytogenes*
*Vibrio cholerae*
*Salmonella typhi*
*Borrelia burgdorferi*
*Porphyromonas gingivalis*
*Klebsiella*

In some embodiments the immunogen elicits an immune response against one of these viruses:
*Orthomyxovirus:* Useful immunogens can be from an influenza A, B or C virus, such as the hemagglutinin, neuraminidase or matrix M2 proteins. Where the immunogen is an influenza A virus hemagglutinin it may be from any subtype *e.g*. H1, H2, H3, H4, H5, H6, H7, H8, H9, H10, H11, H12, H13, H14, H15 or H16.
*Paramyxoviridae* viruses: Viral immunogens include, but are not limited to, those derived from Pneumoviruses (*e.g.* respiratory syncytial virus, RSV), Rubulaviruses (*e.g.* mumps virus), Paramyxoviruses (*e.g.* parainfluenza virus), Metapneumoviruses and Morbilliviruses (*e.g.* measles virus).
*Poxviridae:* Viral immunogens include, but are not limited to, those derived from *Orthopoxvirus* such as *Variola vera,* including but not limited to, *Variola major* and *Variola minor.*
*Picornavirus:* Viral immunogens include, but are not limited to, those derived from Picornaviruses, such as Enteroviruses, Rhinoviruses, Heparnavirus, Cardioviruses and Aphthoviruses. In one embodiment, the enterovirus is a poliovirus *e.g.* a type 1, type 2 and/or type 3 poliovirus. In another embodiment, the enterovirus is an EV71 enterovirus. In another embodiment, the enterovirus is a coxsackie A or B virus.
*Bunyavirus:* Viral immunogens include, but are not limited to, those derived from an *Orthobunyavirus,* such as California encephalitis virus, a *Phlebovirus,* such as Rift Valley Fever virus, or a *Nairovirus,* such as *Crimean-Congo hemorrhagic fever* virus.
*Heparnavirus:* Viral immunogens include, but are not limited to, those derived from a Heparnavirus, such as hepatitis A virus (HAV).
*Filovirus:* Viral immunogens include, but are not limited to, those derived from a filovirus, such as an Ebola virus (including a Zaire, Ivory Coast, Reston or Sudan ebolavirus) or a Marburg virus.
*Togavirus:* Viral immunogens include, but are not limited to, those derived from a Togavirus, such as a Rubivirus, an Alphavirus, or an Arterivirus. This includes rubella virus.
*Flavivirus:* Viral immunogens include, but are not limited to, those derived from a Flavivirus, such as Tick-borne encephalitis (TBE) virus, Dengue (types 1, 2, 3 or 4) virus, Yellow Fever virus, Japanese encephalitis virus, Kyasanur Forest Virus, West Nile encephalitis virus, St. Louis encephalitis virus, Russian spring-summer encephalitis virus, Powassan encephalitis virus.
*Pestivirus:* Viral immunogens include, but are not limited to, those derived from a Pestivirus, such as Bovine viral diarrhea (BVDV), Classical swine fever (CSFV) or Border disease (BDV).
*Hepadnavirus:* Viral immunogens include, but are not limited to, those derived from a Hepadnavirus, such as Hepatitis B virus. A composition can include hepatitis B virus surface antigen (HBsAg).
*Other hepatitis viruses:* A composition can include an immunogen from a hepatitis C virus, delta hepatitis virus, hepatitis E virus, or hepatitis G virus.
*Rhabdovirus*: Viral immunogens include, but are not limited to, those derived from a Rhabdovirus, such as a Lyssavirus (*e.g*. a Rabies virus) and Vesiculovirus (VSV).
*Caliciviridae:* Viral immunogens include, but are not limited to, those derived from Calciviridae, such as Norwalk virus (Norovirus), and Norwalk-like Viruses, such as Hawaii Virus and Snow Mountain Virus.
*Coronavirus:* Viral immunogens include, but are not limited to, those derived from a SARS coronavirus, avian infectious bronchitis (IBV), Mouse hepatitis virus (MHV), and Porcine transmissible gastroenteritis virus (TGEV). The coronavirus immunogen may be a spike polypeptide.
*Retrovirus:* Viral immunogens include, but are not limited to, those derived from an Oncovirus, a Lentivirus (*e.g*. HIV-1 or HIV-2) or a Spumavirus.
*Reovirus:* Viral immunogens include, but are not limited to, those derived from an Orthoreovirus, a Rotavirus, an Orbivirus, or a Coltivirus.
*Parvovirus:* Viral immunogens include, but are not limited to, those derived from Parvovirus B19.
*Herpesvirus:* Viral immunogens include, but are not limited to, those derived from a human herpesvirus, such as, by way of example only, Herpes Simplex Viruses (HSV) (*e.g*. HSV types 1 and 2), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8).
*Papovaviruses:* Viral immunogens include, but are not limited to, those derived from Papillomaviruses and Polyomaviruses. The (human) papillomavirus may be of serotype 1, 2, 4, 5, 6, 8, 11, 13, 16, 18, 31, 33, 35, 39, 41, 42, 47, 51, 57, 58, 63 or 65 *e.g.* from one or more of serotypes 6, 11, 16 and/or 18.
*Adenovirus:* Viral immunogens include those derived from adenovirus serotype 36 (Ad-36).

In some embodiments, the immunogen elicits an immune response against a virus which infects fish, such as: infectious salmon anemia virus (ISAV), salmon pancreatic disease virus (SPDV), infectious pancreatic necrosis virus (IPNV), channel catfish virus (CCV), fish lymphocystis disease virus (FLDV), infectious hematopoietic necrosis virus (IHNV), koi herpesvirus, salmon picorna-like virus (also known as picorna-like virus of atlantic salmon), landlocked salmon virus (LSV), atlantic salmon rotavirus (ASR), trout strawberry disease virus (TSD), coho salmon tumor virus (CSTV), or viral hemorrhagic septicemia virus (VHSV).

Fungal immunogens may be derived from Dermatophytres, including: *Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. album, var. discoides, var. ochraceum, *Trichophyton violaceum,* and/or *Trichophyton faviforme;* or from *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Microsporidia, Encephalitozoon* spp., *Septata intestinalis* and *Enterocytozoon bieneusi;* the less common are *Brachiola* spp, *Microsporidium* spp., *Nosema* spp., *Pleistophora* spp., *Trachipleistophora* spp., *Vittaforma* spp *Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei, Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp,* and *Cladosporium spp.*

In some embodiments the immunogen elicits an immune response against a parasite from the *Plasmodium* genus, such as *P.falciparum, P.vivax, P.malariae* or *P.ovale.* Thus the invention may be used for immunising against malaria. In some embodiments the immunogen elicits an immune response against a parasite from the *Caligidae* family, particularly those from the *Lepeophtheirus* and *Caligus* genera *e.g.* sea lice such as *Lepeophtheirus salmonis* or *Caligus rogercresseyi.*

In some embodiments the immunogen elicits an immune response against: pollen allergens (tree-, herb, weed-, and grass pollen allergens); insect or arachnid allergens (inhalant, saliva and venom allergens, *e.g.* mite allergens, cockroach and midges allergens, hymenopthera venom allergens); animal hair and dandruff allergens (from *e.g.* dog, cat, horse, rat, mouse, *etc*.); and food allergens (*e.g.* a gliadin). Important pollen allergens from trees, grasses and herbs are such originating from the taxonomic orders of Fagales, Oleales, Pinales and platanaceae including, but not limited to, birch (Betula), alder (Alnus), hazel (Corylus), hornbeam (Carpinus) and olive (Olea), cedar (Cryptomeria and Juniperus), plane tree (Platanus), the order of Poales including grasses of the genera Lolium, Phleum, Poa, Cynodon, Dactylis, Holcus, Phalaris, Secale, and Sorghum, the orders of Asterales and Urticales including herbs of the genera Ambrosia, Artemisia, and Parietaria. Other important inhalation allergens are those from house dust mites of the genus Dermatophagoides and Euroglyphus, storage mite *e.g.* Lepidoglyphys, Glycyphagus and Tyrophagus, those from cockroaches, midges and fleas *e.g.* Blatella, Periplaneta, Chironomus and Ctenocepphalides, and those from mammals such as cat, dog and horse, venom allergens including such originating from stinging or biting insects such as those from the taxonomic order of Hymenoptera including bees (*Apidae*), wasps (*Vespidea*)*,* and ants (*Formicoidae*).

In some embodiments the immunogen is a tumor antigen selected from: (a) cancer-testis antigens such as NY-ESO-1, SSX2, SCP1 as well as RAGE, BAGE, GAGE and MAGE family polypeptides, for example, GAGE-1, GAGE-2, MAGE-1, MAGE-2, MAGE-3, MAGE-4, MAGE-5, MAGE-6, and MAGE-12 (which can be used, for example, to address melanoma, lung, head and neck, NSCLC, breast, gastrointestinal, and bladder tumors; (b) mutated antigens, for example, p53 (associated with various solid tumors, *e.g.,* colorectal, lung, head and neck cancer), p21/Ras (associated with, *e.g.,* melanoma, pancreatic cancer and colorectal cancer), CDK4 (associated with, *e.g*., melanoma), MUM1 (associated with, *e.g.,* melanoma), caspase-8 (associated with, *e.g.,* head and neck cancer), CIA 0205 (associated with, *e.g.,* bladder cancer), HLA-A2-R1701, beta catenin (associated with, *e.g.,* melanoma), TCR (associated with, *e.g*., T-cell non-Hodgkins lymphoma), BCR-abl (associated with, *e.g*., chronic myelogenous leukemia), triosephosphate isomerase, KIA 0205, CDC-27, and LDLR-FUT; (c) over-expressed antigens, for example, Galectin 4 (associated with, *e.g*., colorectal cancer), Galectin 9 (associated with, *e.g.,* Hodgkin's disease), proteinase 3 (associated with, *e.g.,* chronic myelogenous leukemia), WT 1 (associated with, *e.g*., various leukemias), carbonic anhydrase (associated with, *e.g.,* renal cancer), aldolase A (associated with, *e.g.,* lung cancer), PRAME (associated with, *e.g.,* melanoma), HER-2/neu (associated with, *e.g.,* breast, colon, lung and ovarian cancer), mammaglobin, alpha-fetoprotein (associated with, *e.g*., hepatoma), KSA (associated with, *e.g.,* colorectal cancer), gastrin (associated with, *e.g.,* pancreatic and gastric cancer), telomerase catalytic protein, MUC-1 (associated with, *e.g*., breast and ovarian cancer), G-250 (associated with, *e.g.,* renal cell carcinoma), p53 (associated with, *e.g.,* breast, colon cancer), and carcinoembryonic antigen (associated with, *e.g*., breast cancer, lung cancer, and cancers of the gastrointestinal tract such as colorectal cancer); (d) shared antigens, for example, melanoma-melanocyte differentiation antigens such as MART-1/Melan A, gp100, MC1R, melanocyte-stimulating hormone receptor, tyrosinase, tyrosinase related protein-1/TRP1 and tyrosinase related protein-2/TRP2 (associated with, *e.g*., melanoma); (e) prostate associated antigens such as PAP, PSA, PSMA, PSH-P1, PSM-P1, PSM-P2, associated with *e.g*., prostate cancer; (f) immunoglobulin idiotypes (associated with myeloma and B cell lymphomas, for example). In certain embodiments, tumor immunogens include, but are not limited to, p15, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KP1, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein/cyclophilin C-associated protein), TAAL6, TAG72, TLP, TPS, and the like.

### Pharmaceutical compositions

Liposomes of the invention are useful as components in pharmaceutical compositions for immunising subjects against various diseases. These compositions will typically include a pharmaceutically acceptable carrier in addition to the liposomes. A thorough discussion of pharmaceutically acceptable carriers is available in reference 31.

A pharmaceutical composition of the invention may include one or more small molecule immunopotentiators. For example, the composition may include a TLR2 agonist (*e.g*. Pam3CSK4), a TLR4 agonist (*e.g.* an aminoalkyl glucosaminide phosphate, such as E6020), a TLR7 agonist (*e.g.* imiquimod), a TLR8 agonist (*e.g.* resiquimod) and/or a TLR9 agonist (*e.g.* IC31). Any such agonist ideally has a molecular weight of <2000Da. In some embodiments such agonist(s) are also encapsulated with the RNA inside liposomes, but in other embodiments they are unencapsulated.

Pharmaceutical compositions of the invention may include the liposomes in plain water (*e.g*. w.f.i.) or in a buffer *e.g.* a phosphate buffer, a Tris buffer, a borate buffer, a succinate buffer, a histidine buffer, or a citrate buffer. Buffer salts will typically be included in the 5-20mM range.

Pharmaceutical compositions of the invention may have a pH between 5.0 and 9.5 *e.g.* between 6.0 and 8.0.

Compositions of the invention may include sodium salts (*e.g*. sodium chloride) to give tonicity. A concentration of 10±2 mg/ml NaCl is typical *e.g.* about 9 mg/ml.

Compositions of the invention may include metal ion chelators. These can prolong RNA stability by removing ions which can accelerate phosphodiester hydrolysis. Thus a composition may include one or more of EDTA, EGTA, BAPTA, pentetic acid, *etc..* Such chelators are typically present at between 10-500µM *e.g.* 0.1mM. A citrate salt, such as sodium citrate, can also act as a chelator, while advantageously also providing buffering activity.

Pharmaceutical compositions of the invention may have an osmolality of between 200 mOsm/kg and 400 mOsm/kg, *e.g.* between 240-360 mOsm/kg, or between 290-310 mOsm/kg.

Pharmaceutical compositions of the invention may include one or more preservatives, such as thiomersal or 2-phenoxyethanol. Mercury-free compositions are preferred, and preservative-free vaccines can be prepared.

Pharmaceutical compositions of the invention are preferably sterile.

Pharmaceutical compositions of the invention are preferably non-pyrogenic e.g. containing <1 EU (endotoxin unit, a standard measure) per dose, and preferably <0.1 EU per dose.

Pharmaceutical compositions of the invention are preferably gluten free.

Pharmaceutical compositions of the invention may be prepared in unit dose form. In some embodiments a unit dose may have a volume of between 0.1-1.0ml *e.g.* about 0.5ml.

The compositions may be prepared as injectables, either as solutions or suspensions. The composition may be prepared for pulmonary administration *e.g.* by an inhaler, using a fine spray. The composition may be prepared for nasal, aural or ocular administration e.g. as spray or drops. Injectables for intramuscular administration are typical.

Compositions comprise an immunologically effective amount of liposomes, as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g*. non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. The liposome and RNA content of compositions of the invention will generally be expressed in terms of the amount of RNA per dose. A preferred dose has ≤100µg RNA (*e.g*. from 10-100µg, such as about 10µg, 25µg, 50µg, 75µg or 100µg). Although expression can be seen at much lower levels (*e.g*. ≤1µg/dose, ≤100ng/dose, ≤10ng/dose, ≤1ng/dose), a minimum dose of 0.1 µg is preferred.

The invention also provides a delivery device (*e.g*. syringe, nebuliser, sprayer, inhaler, dermal patch, *etc*.) containing a pharmaceutical composition of the invention. This device can be used to administer the composition to a vertebrate subject.

Liposomes of the invention do not contain ribosomes.

### Methods of treatment and medical uses

In contrast to the particles disclosed in reference 12, liposomes and pharmaceutical compositions of the invention are for *in vivo* use for eliciting an immune response against an immunogen of interest.

The invention provides a method for raising an immune response in a vertebrate comprising the step of administering an effective amount of a liposome or pharmaceutical composition of the invention. The immune response is preferably protective and preferably involves antibodies and/or cell-mediated immunity. The method may raise a booster response.

The invention also provides a liposome or pharmaceutical composition of the invention for use in a method for raising an immune response in a vertebrate.

The invention also provides the use of a liposome of the invention in the manufacture of a medicament for raising an immune response in a vertebrate.

By raising an immune response in the vertebrate by these uses and methods, the vertebrate can be protected against various diseases and/or infections *e.g.* against bacterial and/or viral diseases as discussed above. The liposomes and compositions are immunogenic, and are more preferably vaccine compositions. Vaccines according to the invention may either be prophylactic (*i.e.* to prevent infection) or therapeutic (*i.e.* to treat infection), but will typically be prophylactic.

The vertebrate is preferably a mammal, such as a human or a large veterinary mammal (*e.g*. horses, cattle, deer, goats, pigs). Where the vaccine is for prophylactic use, the human is preferably a child (*e.g.* a toddler or infant) or a teenager; where the vaccine is for therapeutic use, the human is preferably a teenager or an adult. A vaccine intended for children may also be administered to adults *e.g.* to assess safety, dosage, immunogenicity, *etc.*

Vaccines prepared according to the invention may be used to treat both children and adults. Thus a human patient may be less than 1 year old, less than 5 years old, 1-5 years old, 5-15 years old, 15-55 years old, or at least 55 years old. Preferred patients for receiving the vaccines are the elderly (*e.g.* ≥50 years old, ≥60 years old, and preferably ≥65 years), the young (*e.g.* ≤5 years old), hospitalised patients, healthcare workers, armed service and military personnel, pregnant women, the chronically ill, or immunodeficient patients. The vaccines are not suitable solely for these groups, however, and may be used more generally in a population.

Compositions of the invention will generally be administered directly to a patient. Direct delivery may be accomplished by parenteral injection (*e.g*. subcutaneously, intraperitoneally, intravenously, intramuscularly, intradermally, or to the interstitial space of a tissue; unlike reference 1, intraglossal injection is not typically used with the present invention). Alternative delivery routes include rectal, oral (*e.g.* tablet, spray), buccal, sublingual, vaginal, topical, transdermal or transcutaneous, intranasal, ocular, aural, pulmonary or other mucosal administration. Intradermal and intramuscular administration are two preferred routes. Injection may be via a needle (*e.g.* a hypodermic needle), but needle-free injection may alternatively be used. A typical intramuscular dose is 0.5 ml.

The invention may be used to elicit systemic and/or mucosal immunity, preferably to elicit an enhanced systemic and/or mucosal immunity.

Dosage can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Multiple doses will typically be administered at least 1 week apart (*e.g*. about 2 weeks, about 3 weeks, about 4 weeks, about 6 weeks, about 8 weeks, about 10 weeks, about 12 weeks, about 16 weeks, *etc*.). In one embodiment, multiple doses may be administered approximately 6 weeks, 10 weeks and 14 weeks after birth, e.g. at an age of 6 weeks, 10 weeks and 14 weeks, as often used in the World Health Organisation's Expanded Program on Immunisation ("EPI"). In an alternative embodiment, two primary doses are administered about two months apart, e.g. about 7, 8 or 9 weeks apart, followed by one or more booster doses about 6 months to 1 year after the second primary dose, e.g. about 6, 8, 10 or 12 months after the second primary dose. In a further embodiment, three primary doses are administered about two months apart, e.g. about 7, 8 or 9 weeks apart, followed by one or more booster doses about 6 months to 1 year after the third primary dose, e.g. about 6, 8, 10, or 12 months after the third primary dose.

### Formula (X)

Disclosed are compounds of formula (X) contains a hydrophilic polymer head group linked to a lipid moiety. They can be described as "stealth lipids" and they have formula: 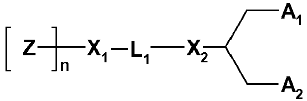 wherein:
[Z]ₙ is a hydrophilic head group component selected from PEG and polymers based on poly(oxazoline), poly(ethylene oxide), poly(vinyl alcohol), poly(glycerol), poly(N-vinylpyrrolidone), poly[N-(2-hydroxypropyl)methacrylamide] and poly(amino acid)s, wherein the polymer may be linear or branched, and wherein the polymer may be optionally substituted;
wherein Z is polymerized by n subunits;
n is a number-averaged degree of polymerization between 10 and 200 units of Z, wherein n is optimized for different polymer types;
L₁ is an optionally substituted C₁₋₁₀ alkylene or C₁₋₁₀ heteroalkylene linker including zero, one or two of an ether (e.g., -O-), ester (e.g., -C(O)O-), succinate (e.g., -O(O)C-CH₂-CH₂-C(O)O-)), carbamate (e.g., -OC(O)-NR'-), carbonate (e.g., -OC(O)O-), urea (e.g., -NRC(O)NR'-), amine (e.g., -NR'-), amide (e.g., -C(O)NR'-), imine (e.g., -C(NR')-), thioether (e.g., -S-), xanthate (e.g., -OC(S)S-), and phosphodiester (e.g., -OP(O)₂O-),
wherein R' is independently selected from -H, -NH-, -NH₂, -O-, -S-, a phosphate or an optionally substituted C₁₋₁₀ alkylene;
X₁ and X₂ are independently selected from a carbon or a heteroatom selected from - NH-, -O-, -S- or a phosphate;
A₁ and A₂ are independently selected from a C₆₋₃₀ alkyl, C₆₋₃₀ alkenyl, and C₆₋₃₀ alkynyl, wherein A₁ and A₂ may be the same or different, or A₁ and A₂ together with the carbon atom to which they are attached form an optionally substituted steroid.

In one embodiment, the compound of formula (X) has formula (X') 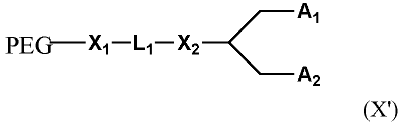 wherein
PEG is a poly(ethylene glycol), wherein the PEG may be linear or branched;
n is a number-averaged degree of polymerization between 70 and 240 units of ethylene oxide;
L₁ is an optionally substituted C₁₋₁₀ heteroalkylene linker containing one or two of an ether, ester, succinate, carbamate, carbonate, urea, amine, amide, imine, thioether, xanthate, and phosphodiester;
X₁ and X₂ are oxygen;
A₁ and A₂ are independently selected from a C₆₋₃₀ alkyl, C₆₋₃₀ alkenyl, and C₆₋₃₀ alkynyl, wherein A₁ and A₂ may be the same or different, or wherein A₁ and A₂ together with the carbon atom to which they are attached form an optionally substituted steroid.

In some embodiments of the invention where a lipid has the formula X', the invention does not encompass lipids where n is a number-averaged degree of polymerization of 200 units of PEG. In other embodiments where a lipid has the formula X', the invention does not encompass lipids where n is a number-averaged degree of polymerization between 190-210 units of PEG. In other embodiments where a lipid has the formula X', the invention does not encompass lipids where n is a number-averaged degree of polymerization above 150 units of PEG or above 130 units of PEG. In some embodiments of the invention where a lipid has the formula X', the invention does not encompass lipids in which n is a number-averaged degree of polymerization between 10 and 200 units of PEG. In some embodiments the invention does not encompass liposomes which include a lipid having formula X'.

The lipids of formulae (X) and (X'), when formulated with cationic lipids to form liposomes, can increase the length of time for which a liposome can exist *in vivo* (*e.g.* in the blood). They can shield the surface of a liposome surface and thereby reduce opsonisation by blood proteins and uptake by macrophages. Further details are in references 32 and 33. In one embodiment, the lipid comprises a group selected from PEG (sometimes referred to as poly(ethylene oxide)) and polymers based on poly(oxazoline), poly(vinyl alcohol), poly(glycerol), poly(N-vinylpyrrolidone), poly[N-(2-hydroxypropyl)methacrylamide] and poly(amino acid)s.

Suitable PEGylated lipids for use with the invention include polyethyleneglycol-diacylglycerol or polyethyleneglycol-diacylglycamide (PEG-DAG) conjugates including those comprising a dialkylglycerol or dialkylglycamide group having alkyl chain length independently comprising from about C4 to about C40 saturated or unsaturated carbon atoms. The dialkylglycerol or dialkylglycamide group can further comprise one or more substituted alkyl groups. The PEGyltaed lipid can be selected from PEG-dilaurylglycerol, PEG-dimyristylglycerol (catalog #GM-020 from NOF), PEG-dipalmitoylglycerol, PEG-disterylglycerol, PEG-dilaurylglycamide, PEG-dimyristylglycamide, PEG-dipalmitoyl-glycamide, and PEG-disterylglycamide, PEG-cholesterol (1-[8'-(Cholest-5-en-3[beta]-oxy)carboxamido-3',6'-dioxaoctanyl]carbamoyl-[omega]-methyl-poly(ethylene glycol), PEG-DMB (3,4-Ditetradecoxylbenzyl-[omega]-methyl-poly(ethylene glycol) ether), 1,2-dimyristoyl-sn-glycero-3-phosphoethanolamine-N-[methoxy (polyethylene glycol)-5000] (catalog #880210P from Avanti Polar Lipids).

### Chemical terms and definitions

### Halo

The term "halogen" (or "halo") includes fluorine, chlorine, bromine and iodine.

### Alkyl, alkylene, alkenyl, alkynyl, cycloalkyl etc.

The terms "alkyl", "alkylene", "alkenyl" and "alkynyl" are used herein to refer to both straight and branched chain acyclic forms. Cyclic analogues thereof are referred to as cycloalkyl, *etc.*

The term "alkyl" includes monovalent, straight or branched, saturated, acyclic hydrocarbyl groups. In one embodiment alkyl is C₁₋₁₀alkyl, in another embodiment C₁₋₆alkyl, in another embodiment C₁₋₄alkyl, such as methyl, ethyl, n-propyl, i-propyl or t-butyl groups.

The term "cycloalkyl" includes monovalent, saturated, cyclic hydrocarbyl groups. In one embodiment cycloalkyl is C₃₋₁₀cycloalkyl, in another embodiment C₃₋₆cycloalkyl such as cyclopentyl and cyclohexyl.

The term "alkoxy" means alkyl-O-.

The term "alkenyl" includes monovalent, straight or branched, unsaturated, acyclic hydrocarbyl groups having at least one carbon-carbon double bond and, in one embodiment, no carbon-carbon triple bonds. In one embodiment alkenyl is C₂₋₁₀alkenyl, in another embodiment C₂₋₆alkenyl, in another embodiment C₂₋₄alkenyl.

The term "cycloalkenyl" includes monovalent, partially unsaturated, cyclic hydrocarbyl groups having at least one carbon-carbon double bond and, in one embodiment, no carbon-carbon triple bonds. In one embodiment cycloalkenyl is C₃₋₁₀cycloalkenyl, in another embodiment C₅₋₁₀cycloalkenyl, e.g. cyclohexenyl or benzocyclohexyl.

The term "alkynyl" includes monovalent, straight or branched, unsaturated, acyclic hydrocarbyl groups having at least one carbon-carbon triple bond and, in one embodiment, no carbon-carbon double bonds. In one embodiment, alkynyl is C₂₋₁₀alkynyl, in another embodiment C₂₋₆alkynyl, in another embodiment C₂₋₄alkynyl.

The term "cycloalkynyl" includes monovalent, partially unsaturated, cyclic hydrocarbyl groups having at least one carbon-carbon triple bond and, in one embodiment, no carbon-carbon double bonds. In one embodiment cycloalkynyl is C₃₋₁₀cycloalkenyl, in another embodiment C₅₋₁₀cycloalkynyl.

The term "alkylene" includes divalent, straight or branched, saturated, acyclic hydrocarbyl groups. In one embodiment alkylene is C₁₋₁₀alkylene, in another embodiment C₁₋₆alkylene, in another embodiment C₁₋₄alkylene, such as methylene, ethylene, n-propylene, i-propylene or t-butylene groups.

The term "alkenylene" includes divalent, straight or branched, unsaturated, acyclic hydrocarbyl groups having at least one carbon-carbon double bond and, in one embodiment, no carbon-carbon triple bonds. In one embodiment alkenylene is C₂₋₁₀alkenylene, in another embodiment C₂₋₆alkenylene, in another embodiment C₂₋₄alkenylene.

The term "alkynylene" includes divalent, straight or branched, unsaturated, acyclic hydrocarbyl groups having at least one carbon-carbon triple bond and, in one embodiment, no carbon-carbon double bonds. In one embodiment alkynylene is C₂₋₁₀alkynylene, in another embodiment C₂₋₆alkynylene, in another embodiment C₂₋₄alkynylene.

### Heteroalkyl etc.

The term "heteroalkyl" includes alkyl groups in which up to six carbon atoms, in one embodiment up to five carbon atoms, in another embodiment up to four carbon atoms, in another embodiment up to three carbon atoms, in another embodiment up to two carbon atoms, in another embodiment one carbon atom, are each replaced independently by O, S(O)_{q}, N, P(O)ᵣ or Si (and preferably O, S(O)_{q} or N), provided at least one of the alkyl carbon atoms remains. The heteroalkyl group may be C-linked or hetero-linked, *i.e.* it may be linked to the remainder of the molecule through a carbon atom or through O, S(O)_{q}, N, P(O)ᵣ or Si.

The term "heterocycloalkyl" includes cycloalkyl groups in which up to six carbon atoms, in one embodiment up to five carbon atoms, in another embodiment up to four carbon atoms, in another embodiment up to three carbon atoms, in another embodiment up to two carbon atoms, in another embodiment one carbon atom, are each replaced independently by O, S(O)_{q} or N, provided at least one of the cycloalkyl carbon atoms remains. Examples of heterocycloalkyl groups include oxiranyl, thiaranyl, aziridinyl, oxetanyl, thiatanyl, azetidinyl, tetrahydrofuranyl, tetrahydrothiophenyl, pyrrolidinyl, tetrahydropyranyl, tetrahydrothiopyranyl, piperidinyl, 1,4-dioxanyl, 1,4-oxathianyl, morpholinyl, 1,4-dithianyl, piperazinyl, 1,4-azathianyl, oxepanyl, thiepanyl, azepanyl, 1,4-dioxepanyl, 1,4-oxathiepanyl, 1,4-oxaazepanyl, 1,4-dithiepanyl, 1,4-thieazepanyl and 1,4-diazepanyl. The heterocycloalkyl group may be C-linked or N-linked, *i.e.* it may be linked to the remainder of the molecule through a carbon atom or through a nitrogen atom.

The term "heteroalkenyl" includes alkenyl groups in which up to three carbon atoms, in one embodiment up to two carbon atoms, in another embodiment one carbon atom, are each replaced independently by O, S(O)_{q} or N, provided at least one of the alkenyl carbon atoms remains. The heteroalkenyl group may be C-linked or hetero-linked, *i.e.* it may be linked to the remainder of the molecule through a carbon atom or through O, S(O)_{q} or N.

The term "heterocycloalkenyl" includes cycloalkenyl groups in which up to three carbon atoms, in one embodiment up to two carbon atoms, in another embodiment one carbon atom, are each replaced independently by O, S(O)_{q} or N, provided at least one of the cycloalkenyl carbon atoms remains. Examples of heterocycloalkenyl groups include 3,4-dihydro-2H-pyranyl, 5-6-dihydro-2H-pyranyl, 2H-pyranyl, 1,2,3,4-tetrahydropyridinyl and 1,2,5,6-tetrahydropyridinyl. The heterocycloalkenyl group may be C-linked or N-linked, *i.e.* it may be linked to the remainder of the molecule through a carbon atom or through a nitrogen atom.

The term "heteroalkynyl" includes alkynyl groups in which up to three carbon atoms, in one embodiment up to two carbon atoms, in another embodiment one carbon atom, are each replaced independently by O, S(O)_{q} or N, provided at least one of the alkynyl carbon atoms remains. The heteroalkynyl group may be C-linked or hetero-linked, *i.e.* it may be linked to the remainder of the molecule through a carbon atom or through O, S(O)_{q} or N.

The term "heterocycloalkynyl" includes cycloalkynyl groups in which up to three carbon atoms, in one embodiment up to two carbon atoms, in another embodiment one carbon atom, are each replaced independently by O, S(O)_{q} or N, provided at least one of the cycloalkynyl carbon atoms remains. The heterocycloalkenyl group may be C-linked or N-linked, *i.e.* it may be linked to the remainder of the molecule through a carbon atom or through a nitrogen atom.

The term "heteroalkylene" includes alkylene groups in which up to three carbon atoms, in one embodiment up to two carbon atoms, in another embodiment one carbon atom, are each replaced independently by O, S(O)_{q} or N, provided at least one of the alkylene carbon atoms remains.

The term "heteroalkenylene" includes alkenylene groups in which up to three carbon atoms, in one embodiment up to two carbon atoms, in another embodiment one carbon atom, are each replaced independently by O, S(O)_{q} or N, provided at least one of the alkenylene carbon atoms remains.

The term "heteroalkynylene" includes alkynylene groups in which up to three carbon atoms, in one embodiment up to two carbon atoms, in another embodiment one carbon atom, are each replaced independently by O, S(O)_{q} or N, provided at least one of the alkynylene carbon atoms remains.

### Aryl

The term "aryl" includes monovalent, aromatic, cyclic hydrocarbyl groups, such as phenyl or naphthyl (e.g. 1-naphthyl or 2-naphthyl). In general, the aryl groups may be monocyclic or polycyclic fused ring aromatic groups. Preferred aryl are C₆-C₁₄aryl.

Other examples of aryl groups are monovalent derivatives of aceanthrylene, acenaphthylene, acephenanthrylene, anthracene, azulene, chrysene, coronene, fluoranthene, fluorene, as-indacene, s-indacene, indene, naphthalene, ovalene, perylene, phenalene, phenanthrene, picene, pleiadene, pyrene, pyranthrene and rubicene.

The term "arylalkyl" means alkyl substituted with an aryl group, e.g. benzyl.

The term "arylene" includes divalent aromatic, cyclic hydrocarbyl groups, such as phenylene. In general, the arylene groups may be monocyclic or polycyclic fused ring aromatic groups. Preferred arylene are C₆-C₁₄arylene. Other examples of arylene groups are divalent derivatives of aceanthrylene, acenaphthylene, acephenanthrylene, anthracene, azulene, chrysene, coronene, fluoranthene, fluorene, as-indacene, s-indacene, indene, naphthalene, ovalene, perylene, phenalene, phenanthrene, picene, pleiadene, pyrene, pyranthrene and rubicene.

### Heteroaryl

The term "heteroaryl" includes monovalent, heteroaromatic, cyclic hydrocarbyl groups additionally containing one or more heteroatoms independently selected from O, S, N and NR^{N}, where R^{N} is defined below (and in one embodiment is H or alkyl (*e.g.* C₁₋₆alkyl)).

In general, the heteroaryl groups may be monocyclic or polycyclic (*e.g*. bicyclic) fused ring heteroaromatic groups. In one embodiment, heteroaryl groups contain 5-13 ring members (preferably 5-10 members) and 1, 2, 3 or 4 ring heteroatoms independently selected from O, S, N and NR^{N}. In one embodiment, a heteroaryl group may be 5, 6, 9 or 10 membered, e.g. 5-membered monocyclic, 6-membered monocyclic, 9-membered fused-ring bicyclic or 10-membered fused-ring bicyclic.

Monocyclic heteroaromatic groups include heteroaromatic groups containing 5-6 ring members and 1, 2, 3 or 4 heteroatoms selected from O, S, N or NR^{N}.

In one embodiment, 5-membered monocyclic heteroaryl groups contain 1 ring member which is an -NR^{N}- group, an -O- atom or an -S- atom and, optionally, 1-3 ring members (*e.g*. 1 or 2 ring members) which are =N- atoms (where the remainder of the 5 ring members are carbon atoms).

Examples of 5-membered monocyclic heteroaryl groups are pyrrolyl, furanyl, thiophenyl, pyrazolyl, imidazolyl, isoxazolyl, oxazolyl, isothiazolyl, thiazolyl, 1,2,3 triazolyl, 1,2,4 triazolyl, 1,2,3 oxadiazolyl, 1,2,4 oxadiazolyl, 1,2,5 oxadiazolyl, 1,3,4 oxadiazolyl, 1,3,4 thiadiazolyl, pyridyl, pyrimidinyl, pyridazinyl, pyrazinyl, 1,3,5 triazinyl, 1,2,4 triazinyl, 1,2,3 triazinyl and tetrazolyl.

Examples of 6-membered monocyclic heteroaryl groups are pyridinyl, pyridazinyl, pyrimidinyl and pyrazinyl.

In one embodiment, 6-membered monocyclic heteroaryl groups contain 1 or 2 ring members which are =N- atoms (where the remainder of the 6 ring members are carbon atoms).

Bicyclic heteroaromatic groups include fused-ring heteroaromatic groups containing 9-13 ring members and 1, 2, 3, 4 or more heteroatoms selected from O, S, N or NR^{N}.

In one embodiment, 9-membered bicyclic heteroaryl groups contain 1 ring member which is an -NR^{N}- group, an -O- atom or an -S- atom and, optionally, 1-3 ring members (*e.g*. 1 or 2 ring members) which are =N- atoms (where the remainder of the 9 ring members are carbon atoms).

Examples of 9-membered fused-ring bicyclic heteroaryl groups are benzofuranyl, benzothiophenyl, indolyl, benzimidazolyl, indazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, isoindolyl, indazolyl, purinyl, indolininyl, imidazo[1,2-a]pyridinyl, imidazo[1,5-a]pyridinyl, pyrazolo[1,2-a]pyridinyl, pyrrolo[1,2-b]pyridazinyl and imidazo[1,2-c]pyrimidinyl.

In one embodiment, 10-membered bicyclic heteroaryl groups contain 1-3 ring members which are =N- atoms (where the remainder of the 10 ring members are carbon atoms).

Examples of 10-membered fused-ring bicyclic heteroaryl groups are quinolinyl, isoquinolinyl, cinnolinyl, quinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

The term "heteroarylalkyl" means alkyl substituted with a heteroaryl group.

The term "heteroarylene" includes divalent heteroaromatic, cyclic hydrocarbyl groups additionally containing one or more heteroatoms independently selected from O, S, N and NR^{N}, where R^{N} is defined below (and in one embodiment is H or alkyl (*e.g*. C₁₋₆alkyl)). In general, the heteroarylene groups may be monocyclic or polycyclic (*e.g*. bicyclic) fused ring heteroaromatic groups. In one embodiment, heteroarylene groups contain 5-13 ring members (preferably 5-10 members) and 1, 2, 3 or 4 ring heteroatoms independently selected from O, S, N and NR^{N}. In one embodiment, a heteroarylene group may be 5, 6, 9 or 10 membered, e.g. 5-membered monocyclic, 6-membered monocyclic, 9-membered fused-ring bicyclic or 10-membered fused-ring bicyclic. The term "heteroarylene" includes divalent derivatives of each of the heteroaryl groups discussed above.

The terms "aryl", "aromatic", "heteroaryl" and "heteroaromatic" also include groups that are partially reduced. Thus, for example, "heteroaryl" includes fused species in which one of the rings has been reduced to a saturated ring (e.g. 1,2,3,4-tetrahydro-1,8-naphthyridin-2-yl).

### General

Unless indicated explicitly otherwise, where combinations of groups are referred to herein as one moiety, *e.g.* arylalkyl, the last mentioned group contains the atom by which the moiety is attached to the rest of the molecule.

Where reference is made to a carbon atom of an alkyl group or other group being replaced by O, S(O)_{q}, N or P(O)ᵣ, what is intended is that: 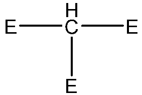 is replaced by 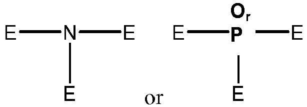 (wherein E cannot be H);
-CH= is replaced by -N= or -P(O)ᵣ=;
≡C-H is replaced by ≡N or ≡P(O)ᵣ; or
-CH₂- is replaced by -O-, -S(O)_{q}-, -NR^{N}- or -P(O)ᵣR^{N}-, where R^{N} is H or optionally substituted C₁₋₆alkyl, C₁₋₆heteroalkyl, C₃₋₆cycloalkyl, C₃₋₆heterocycloalkyl, C₂₋₆alkenyl, C₂₋₆heteroalkenyl, C₃₋₆cycloalkenyl, C₃₋₆heterocycloalkenyl, phenyl, or heteroaryl containing 5 or 6 ring members. R^{N} is preferably H, C₁₋₆alkyl or C₃₋₆cycloalkyl.
q is independently 0, 1 or 2. In one embodiment, q is 0.
r is independently 0 or 1. In one embodiment, r is 0.

Where reference is made to a carbon atom being replaced by Si, what is intended is that the carbon atom is swapped for a silicon atom but that the bonds otherwise remain the same. Thus, for example, -CH₂- is replaced by -SiH₂-; -CH= is replaced by -SiH=; and ≡C-H is replaced by ≡Si-H.

By way of clarification, in relation to the above mentioned heteroatom containing groups (such as heteroalkyl *etc*.), where a numerical of carbon atoms is given, for instance C₃₋₆heteroalkyl, what is intended is a group based on C₃₋₆alkyl in which one or more of the 3-6 chain carbon atoms is replaced by O, S(O)_{q} or N. Accordingly, a C₃₋₆heteroalkyl group would, for example, contain less than 3-6 chain carbon atoms. As another example, a pyridyl group would be classed as a C₆ heteroaryl group even though it contains 5 carbon atoms.

### Substitution

Groups of the compounds used in the invention (*e.g*. alkyl, cycloalkyl, alkoxy, alkenyl, cycloalkenyl, alkynyl, alkylene, alkenylene, heteroalkyl, heterocycloalkyl, heteroalkenyl, heterocycloalkenyl, heteroalkynyl, heteroalkylene, heteroalkenylene aryl, arylalkyl, arylheteroalkyl, heteroaryl, heteroarylalkyl or heteroarylheteroalkyl groups *etc*.) may be substituted or unsubstituted, in one embodiment unsubstituted. Typically, substitution involves the notional replacement of a hydrogen atom with a substituent group, or two hydrogen atoms in the case of substitution by =O.

Where substituted, there will generally be 1 to 5 substituents on each group, in one embodiment 1 to 3 substituents, in one embodiment 1 or 2 substituents, in one embodiment 1 substituent. One embodiment includes more than one substituent on the same atom, e.g. an acetal group.

In one embodiment, the substituent(s) is/are independently Sub¹ or Sub² (in one embodiment Sub²) wherein:
Sub¹ is independently halogen, trihalomethyl, trihaloethyl, -NO₂, -CN , -N⁺(R^{s})₂O⁻, -CO₂H, -CO₂R^{s}, -SO₃H, -SOR^{s}, -SO₂R^{s}, -SO₃R^{s}, -OC(=O)OR^{s}, -C(=O)H,-C(=O)R^{s},-OC(=O) R^{s},=O, -NR^{s}₂, -C(=O)NH₂, -C(=O)NR^{s}₂, -N(R^{s})C(=O)OR^{s}, -N(R^{s})C(=O)NR^{s}₂, -OC(=O)NR^{s}₂, -N(R^{s})C(=O )R^{s}, -C(=S)NR^{s}₂, -NR^{s}C(=S)R^{s}, -SO₂NR^{s}₂, -NR^{s}SO₂R^{s}, -N(R^{s})C(=S)NR^{s}₂, -N(R^{s})SO₂NR^{s}₂, -R^{s} or -Z^{s}R^{s}, wherein;
   Z^{s} is independently O, S or NR^{s};
   R^{s} is independently H or C₁₋₆alkyl, C₁₋₆heteroalkyl, -(Alk^{a})_{f}-C₃₋₆cycloalkyl, -(Alk^{a})_{f}-C₃₋₆heterocycloalkyl, C₂₋₆alkenyl, C₂₋₆heteroalkenyl, -(Alk^{a})_{f}-C₃₋₆cycloalkenyl, -(Alk^{a})_{f}-C₃₋₆heterocycloalkenyl, C₂₋₆alkynyl, C₂₋₆heteroalkynyl, -(Alk^{a})_{f}-C₆₋₁₄aryl, -(Alk^{a})_{f}-C₆₋₁₄aryl or -(Alk^{a})_{f}-heteroaryl (where heteroaryl contains 5-13 ring members), where
      f is 0 or 1;
      Alk^{a} is C₁₋₆alkylene or C₁₋₆heteroalkylene; and
      R^{s} is optionally substituted itself (in one embodiment unsubstituted) by 1 to 3 substituents Sub²;
Sub² is independently halogen, trihalomethyl, trihaloethyl, -NO₂, -CN, -N⁺(C₁₋₆alkyl)₂O⁻, -CO₂H , -CO₂C₁₋₆alkyl, -SO₃H, -SOC₁₋₆alkyl, -SO₂C₁₋₆alkyl, -SO₃C₁₋₆alkyl, -OC(=O)OC₁₋₆alkyl, -C(=O)H, -C(=O )C₁₋₆alkyl, -OC(=O)C₁₋₆alkyl, =O, -N(C₁₋₆alkyl)₂, -C(=O)NH₂, -C(=O)N(C₁₋₆alkyl)₂, -N(C₁₋₆alkyl)C (=O)O(C₁₋₆alkyl), -N(C₁₋₆alkyl)C(=O)N(C₁₋₆alkyl)₂, -OC(=O)N(C₁₋₆alkyl)₂, -N(C₁₋₆alkyl)C(=O)C₁₋₆alkyl, -C(=S)N(C₁₋₆alkyl)₂, -N(C₁₋₆alkyl)C(=S)C₁₋₆alkyl, -SO₂N(C₁₋₆alkyl)₂, -N(C₁₋₆alkyl)SO₂C₁₋₆alkyl, -N(C₁₋₆al kyl)C(=S)N(C₁₋₆alkyl)₂, -N(C₁₋₆alkyl)SO₂N(C₁₋₆alkyl)₂, -C₁₋₆alkyl, -C₁₋₆heteroalkyl, -C₃₋₆cycloalkyl, -C₃₋₆h eterocycloalkyl, -C₂₋₆alkenyl, -C₂₋₆heteroalkenyl, -C₃₋₆cycloalkenyl, -C₃₋₆heterocycloalkenyl, -C₂₋₆alkynyl, -C₂₋₆heteroalkynyl, -C₆₋₁₄aryl, -C₅₋₁₃heteroaryl, -Z^{t}-C₁₋₆alkyl, -Z^{t}-C₃₋₆cycloalkyl, -Z^{t}-C₂₋₆alkenyl,-Z^{t}-C₃₋₆cycloalkenyl, or -Z^{t}-C₂₋₆alkynyl; and
   Z^{t} is independently O, S, NH or N(C₁₋₆alkyl).

While R^{s} in Sub¹ can be optionally substituted by 1 to 3 substituents Sub², Sub² is unsubstituted. However, in one embodiment, R^{s} is unsubstituted.

In one embodiment, R^{s} is H or C₁₋₆alkyl, optionally substituted by 1 to 3 substituents Sub².

In one embodiment, Sub² is independently halogen, trihalomethyl, trihaloethyl, -NO₂, -CN, -N⁺(C₁₋₆alkyl)₂O⁻, -CO₂H, -SO₃H, -SOC₁₋₆alkyl, -SO₂C₁₋₆alkyl, -C(=O)H, -C (=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl,-Z^{t}-C₁₋₆alkyl or -Z^{t}-C₃₋₆cycloalkyl.

In one embodiment, where the substituted group is acyclic (*e.g.* alkyl, heteroalkyl, alkenyl *etc*.), Sub¹ is not -R^{s} and Sub² is not -C₁₋₆alkyl, -C₁₋₆heteroalkyl, -C₂₋₆alkenyl, -C₂₋₆heteroalkenyl, -C₂₋₆alkynyl or -C₂₋₆heteroalkynyl.

Where a group other than Sub² has at least 2 positions which may be substituted, the group may be substituted by both ends of an alkylene, alkenylene, alkynylene, heteroalkylene, heteroalkenylene or heteroalkynylene chain (in one embodiment containing 1 to 6 atoms, in a further embodiment 3 to 6 atoms, and in a further embodiment 3 or 4 atoms) to form a cyclic moiety. That chain is optionally substituted by 1 to 3 substituents Sub². In one embodiment that chain is not substituted. Thus, the terms optionally substituted "cycloalkyl", "cycloalkenyl", "cycloalkynyl", "heterocycloalkyl", "heterocycloalkenyl", "heterocycloalkynyl", "aryl" and "heteroaryl" include fused species. E.g. "optionally substituted cycloalkyl" includes a species in which two cycloalkyl rings are fused, and "optionally substituted heteroaryl" includes a species in which a heterocycloalkyl ring is fused to the aromatic ring (e.g. 5,6,7,8-tetrahydro-1,8-naphthyridin-2-yl).

Where a group other than Sub² has an atom which may be substituted twice, that atom may be substituted by both ends of an alkylene, alkenylene, alkynylene, heteroalkylene, heteroalkenylene or heteroalkynylene chain (in one embodiment containing 2 to 8 atoms, in a further embodiment 3 to 6 atoms, and in a further embodiment 4 or 5 atoms) to form a cyclic moiety. That chain is optionally substituted by 1 to 3 substituents Sub². In one embodiment that chain is not substituted. Thus, the terms optionally substituted "cycloalkyl", "cycloalkenyl", "cycloalkynyl", "heterocycloalkyl", "heterocycloalkenyl", "heterocycloalkynyl", "aryl" and "heteroaryl" include spiro species.

By way of clarification, when a group has a heteroatom, a substituent may be bonded to the heteroatom. Thus, for example, "optionally substituted heteroalkyl" includes -CH₂-N(Sub¹)-CH₂-, -CH(Sub¹)-NH-CH₂- and -CH(Sub¹)-N(Sub¹)-CH₂- etc.

### Modifier terms

When a list is preceded by a modifier, it is intended that the modifier is to be understood as applying to each of the items in the list. For example, the phrase "optionally substituted C₃₋₂₀-heterocycloalkyl, C₃₋₂₀-heterocycloalkenyl, C₃₋₂₀-heterocycloalkynyl or C₅₋₂₀-heteroaryl group" means that each of the four items in the list, namely the C₃₋₂₀-heterocycloalkyl group, the C₃₋₂₀-heterocycloalkenyl group, the C₃₋₂₀-heterocycloalkynyl group and the C₆₋₂₀-heteroaryl group, may be optionally substituted.

When a group is characterised by a first modifier and then, later on, the same group is characterised by a subsequent modifier, what is meant is that the group is characterised by both modifiers simultaneously. For example, if a group is described as a "C₃₋₂₀-heterocycloalkynyl" (the first modifier) group and then later the same group is described as a "C₅₋₁₆" (the subsequent modifier) group, what is meant is a C₅₋₁₆ heterocycloalkynyl group.

### Steroids

As used herein, the term "steroid" refers to any group comprising the following structure (which structure is referred to herein as the "steroid skeleton").

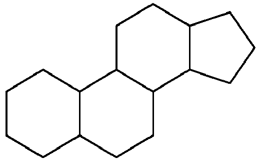

Purely for the purposes of illustration, the steroid skeleton has been drawn above as fully saturated. The term steroid, however, is also intended to cover instances where there is unsaturation in the steroid skeleton. For example, the term steroid covers a group which comprises the fully unsaturated (mancude) basic skeleton, 15*H*-cyclopenta[*a*]phenanthrene:

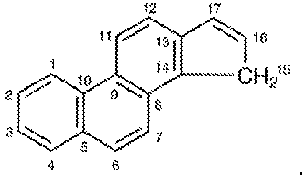

The term steroid also covers a group which comprises a partially unsaturated steroid skeleton.

The term steroid also covers "seco" derivatives of the steroid skeleton, i.e. groups in which ring cleavage has been effected; "nor" and "homo" derivatives of the steroid skeleton which involve ring contraction and expansion, respectively (see Systemic Nomenclature of Organic Chemistry, by D. Hellwinkel, published by Springer, 2001, ISBN: 3-540-41138-0, page 203 for "seco" and page 204 for "nor" and "homo"). In one embodiment, however, such seco derivatives are not encompassed by the term "steroid". In another embodiment, such nor derivatives are not encompassed by the term "steroid". In another embodiment, such homo derivatives are not encompassed by the term "steroid". Thus in one embodiment, such seco, nor and homo derivatives are not encompassed by the term "steroid".

The term steroid also covers instances where one or more of the carbon atoms in the structure labelled steroid skeleton is replaced by a heteroatom. In one such embodiment, up to six carbon atoms, in one embodiment up to five carbon atoms, in another embodiment up to four carbon atoms, in another embodiment up to three carbon atoms, in another embodiment up to two carbon atoms, in another embodiment one carbon atom, are each replaced independently by O, S(O)_{q}, N, P(O)ᵣ or Si (and preferably O, S(O)_{q} or N). In one embodiment, however, the term "steroid" comprises species in which the "steroid basic skeleton" contains no heteroatoms.

A steroid ring system is numbered according to the convention set out below.

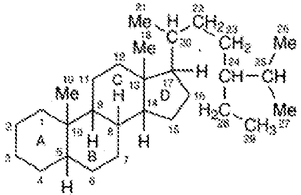

The term steroid encompasses sterols, steroid hormones, bile acids and salts of bile acids. A sterol is any steroid with a hydroxyl group at the 3-position of the A-ring.

### Unsaturation

In accordance with standard use, the omega-3 position refers to the third bond from the (methyl) terminal of the chain; the omega-6 position refers to the sixth bond from the (methyl) terminal of the chain and the omega-9 position refers to the ninth bond from the (methyl) terminal of the chain.

### General

The practice of the present invention will employ, unless otherwise indicated, conventional methods of chemistry, biochemistry, molecular biology, immunology and pharmacology, within the skill of the art. Such techniques are explained fully in the literature. See, *e.g.,* references 34-40, *etc.*

The term "comprising" encompasses "including" as well as "consisting" *e.g.* a composition "comprising" X may consist exclusively of X or may include something additional *e.g.* X + Y.

The term "about" in relation to a numerical value x is optional and means, for example, *x*±10%.

The word "substantially" does not exclude "completely" *e.g.* a composition which is "substantially free" from Y may be completely free from Y. Where necessary, the word "substantially" may be omitted from the definition of the invention.

References to charge, to cations, to anions, to zwitterions, *etc.,* are taken at pH 7.

TLR3 is the Toll-like receptor 3. It is a single membrane-spanning receptor which plays a key role in the innate immune system. Known TLR3 agonists include poly(I:C). "TLR3" is the approved HGNC name for the gene encoding this receptor, and its unique HGNC ID is HGNC: 11849. The RefSeq sequence for the human TLR3 gene is GI:2459625.

TLR7 is the Toll-like receptor 7. It is a single membrane-spanning receptor which plays a key role in the innate immune system. Known TLR7 agonists include e.g. imiquimod. "TLR7" is the approved HGNC name for the gene encoding this receptor, and its unique HGNC ID is HGNC:15631. The RefSeq sequence for the human TLR7 gene is GI:67944638.

TLR8 is the Toll-like receptor 8. It is a single membrane-spanning receptor which plays a key role in the innate immune system. Known TLR8 agonists include e.g. resiquimod. "TLR8" is the approved HGNC name for the gene encoding this receptor, and its unique HGNC ID is HGNC:15632. The RefSeq sequence for the human TLR8 gene is GI:20302165.

The RIG-I-like receptor ("RLR") family includes various RNA helicases which play key roles in the innate immune system[41]. RLR-1 (also known as RIG-I or retinoic acid inducible gene I) has two caspase recruitment domains near its N-terminus. The approved HGNC name for the gene encoding the RLR-1 helicase is "DDX58" (for DEAD (Asp-Glu-Ala-Asp) box polypeptide 58) and the unique HGNC ID is HGNC:19102. The RefSeq sequence for the human RLR-1 gene is GI:77732514. RLR-2 (also known as MDA5 or melanoma differentiation-associated gene 5) also has two caspase recruitment domains near its N-terminus. The approved HGNC name for the gene encoding the RLR-2 helicase is "IFIH1" (for interferon induced with helicase C domain 1) and the unique HGNC ID is HGNC:18873. The RefSeq sequence for the human RLR-2 gene is GI: 27886567. RLR-3 (also known as LGP2 or laboratory of genetics and physiology 2) has no caspase recruitment domains. The approved HGNC name for the gene encoding the RLR-3 helicase is "DHX58" (for DEXH (Asp-Glu-X-His) box polypeptide 58) and the unique HGNC ID is HGNC:29517. The RefSeq sequence for the human RLR-3 gene is GI:149408121.

PKR is a double-stranded RNA-dependent protein kinase. It plays a key role in the innate immune system. "EIF2AK2" (for eukaryotic translation initiation factor 2-alpha kinase 2) is the approved HGNC name for the gene encoding this enzyme, and its unique HGNC ID is HGNC:9437. The RefSeq sequence for the human PKR gene is GI:208431825.

### BRIEF DESCRIPTION OF DRAWINGS

Figure 1 shows a gel with stained RNA. Lanes show (1) markers (2) naked replicon (3) replicon after RNase treatment (4) replicon encapsulated in liposome (5) liposome after RNase treatment (6) liposome treated with RNase then subjected to phenol/chloroform extraction.
Figure 2 shows a gel with stained RNA. Lanes show (1) markers (2) naked replicon (3) replicon encapsulated in liposome (4) liposome treated with RNase then subjected to phenol/chloroform extraction.
Figure 3 shows protein expression (as relative light units, RLU) at days 1, 3 and 6 after delivery of RNA in liposomes with PEGs of different lengths: 1kDa (triangles); 2kDa (circles); 3kDa (squares).
Figure 4 shows protein expression at days 1, 3 and 6 after delivery of RNA as a virion-packaged replicon (squares), as naked RNA (diamonds), or in liposomes (+ = 0.1µg, x = 1µg).
Figure 5 shows log₁₀ F-specific IgG titers in BALB/c mice. The 5 groups had PEG lengths, from left to right, of 1, 2, 3, 5 or 10kDa. Circles show titers 2 weeks after 1 injection; triangles show titers 2 weeks after a 2nd injection; bars show the mean for the two titers.

### MODES FOR CARRYING OUT THE INVENTION

### RNA replicons

Various replicons are used below. In general these are based on a hybrid alphavirus genome with non-structural proteins from venezuelan equine encephalitis virus (VEEV), a packaging signal from VEEV, and a 3' UTR from Sindbis virus or a VEEV mutant. The replicon is about 10kb long and has a poly-A tail.

Plasmid DNA encoding alphavirus replicons (named: pT7-mVEEV-FL.RSVF or A317; pT7-mVEEV-SEAP or A306; pSP6-VCR-GFP or A50) served as a template for synthesis of RNA *in vitro.* The replicons contain the alphavirus genetic elements required for RNA replication but lack those encoding gene products necessary for particle assembly; the structural proteins are instead replaced by a protein of interest (either a reporter, such as SEAP or GFP, or an immunogen, such as full-length RSV F protein) and so the replicons are incapable of inducing the generation of infectious particles. A bacteriophage (T7 or SP6) promoter upstream of the alphavirus cDNA facilitates the synthesis of the replicon RNA *in vitro* and a hepatitis delta virus (HDV) ribozyme immediately downstream of the poly(A)-tail generates the correct 3'-end through its self-cleaving activity.

Following linearization of the plasmid DNA downstream of the HDV ribozyme with a suitable restriction endonuclease, run-off transcripts were synthesized in vitro using T7 or SP6 bacteriophage derived DNA-dependent RNA polymerase. Transcriptions were performed for 2 hours at 37°C in the presence of 7.5 mM (T7 RNA polymerase) or 5 mM (SP6 RNA polymerase) of each of the nucleoside triphosphates (ATP, CTP, GTP and UTP) following the instructions provided by the manufacturer (Ambion). Following transcription the template DNA was digested with TURBO DNase (Ambion). The replicon RNA was precipitated with LiCl and reconstituted in nuclease-free water. Uncapped RNA was capped post-transcriptionally with Vaccinia Capping Enzyme (VCE) using the ScriptCap m7G Capping System (Epicentre Biotechnologies) as outlined in the user manual; replicons capped in this way are given the "v" prefix *e.g.* vA317 is the A317 replicon capped by VCE. Post-transcriptionally capped RNA was precipitated with LiCl and reconstituted in nuclease-free water. The concentration of the RNA samples was determined by measuring OD₂₆₀ₙₘ. Integrity of the *in vitro* transcripts was confirmed by denaturing agarose gel electrophoresis.

### Liposomal encapsulation

RNA was encapsulated in liposomes made essentially by the method of references 9 and 42. Briefly, lipids were dissolved in ethanol, a RNA replicon was dissolved in buffer, and these were mixed with buffer followed by equilibration. The mixture was diluted with buffer then filtered. The resulting product contained liposomes, with high encapsulation efficiency. The liposomes were made of 10% DSPC (zwitterionic), 40% DlinDMA (cationic), 48% cholesterol and 2% PEG-conjugated DMG. These proportions refer to the % moles in the total liposome.

DlinDMA (1,2-dilinoleyloxy-N,N-dimethyl-3-aminopropane) was synthesized using the procedure of reference 4. DSPC (1,2-Diastearoyl-sn-glycero-3-phosphocholine) was purchased from Genzyme. Cholesterol was obtained from Sigma-Aldrich. PEG-conjugated DMG (1,2-dimyristoyl-sn-glycero-3-phosphoethanolamine-N-[methoxy(polyethylene glycol), ammonium salt), DOTAP (1,2-dioleoyl-3-trimethylammonium-propane, chloride salt) and DC-chol (3β-[N-(N',N'-dimethylaminoethane)-carbamoyl]cholesterol hydrochloride) were from Avanti Polar Lipids.

In some liposomes alternative cationic lipids were used instead of DlinDMA *e.g.* RV05 or RV17:

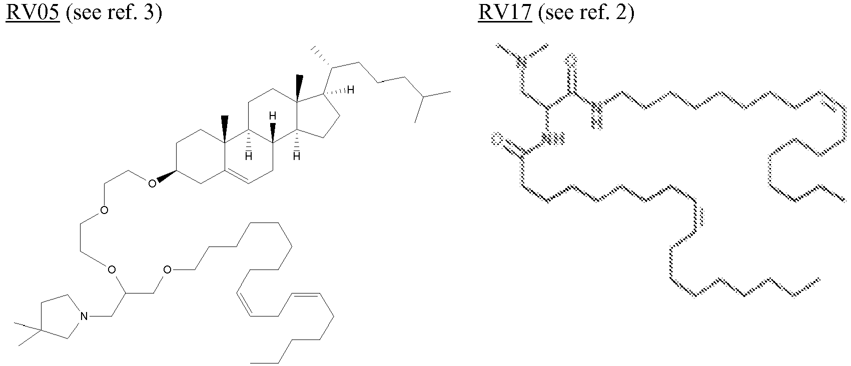

In general, eight different methods have been used for preparing liposomes. These are referred to in the text as methods (A) to (H) and they differ mainly in relation to filtration and TFF steps. Details are as follows:
(A) Fresh lipid stock solutions in ethanol were prepared. 37 mg of DlinDMA, 11.8 mg of DSPC, 27.8 mg of Cholesterol and 8.07 mg of PEG-DMG 2000 were weighed and dissolved in 7.55 mL of ethanol. The freshly prepared lipid stock solution was gently rocked at 37°C for about 15 min to form a homogenous mixture. Then, 755 µL of the stock was added to 1.245 mL ethanol to make a working lipid stock solution of 2 mL. This amount of lipids was used to form liposomes with 250 µg RNA. A 2 mL working solution of RNA was also prepared from a stock solution of ∼ 1µg/µL in 100 mM citrate buffer (pH 6). Three 20 mL glass vials (with stir bars) were rinsed with RNase Away solution (Molecular BioProducts) and washed with plenty of MilliQ water before use to decontaminate the vials of RNases. One of the vials was used for the RNA working solution and the others for collecting the lipid and RNA mixes (as described later). The working lipid and RNA solutions were heated at 37°C for 10 min before being loaded into 3cc luer-lok syringes. 2 mL of citrate buffer (pH 6) was loaded in another 3 cc syringe. Syringes containing RNA and the lipids were connected to a T mixer (PEEK™ 500 µm ID junction, Idex Health Science) using FEP tubing (fluorinated ethylene-propylene; al FEP tubing has a 2mm internal diameter x 3mm outer diameter, supplied by Idex Health Science). The outlet from the T mixer was also FEP tubing. The third syringe containing the citrate buffer was connected to a separate piece of FEP tubing. All syringes were then driven at a flow rate of 7 mL/min using a syringe pump. The tube outlets were positioned to collect the mixtures in a 20 mL glass vial (while stirring). The stir bar was taken out and the ethanol/aqueous solution was allowed to equilibrate to room temperature for 1 hour. 4 ml of the mixture was loaded into a 5 cc syringe, which was connected to a piece of FEP tubing and in another 5 cc syringe connected to an equal length of FEP tubing, an equal amount of 100 mM citrate buffer (pH 6) was loaded. The two syringes were driven at 7mL/min flow rate using the syringe pump and the final mixture collected in a 20 mL glass vial (while stirring). Next, the mixture collected from the second mixing step (liposomes) were passed through a Mustang Q membrane (an anion-exchange support that binds and removes anionic molecules, obtained from Pall Corporation). Before passing the liposomes, 4 mL of 1 M NaOH, 4 mL of 1 M NaCl and 10 mL of 100 mM citrate buffer (pH 6) were successively passed through the Mustang membrane. Liposomes were warmed for 10 min at 37°C before passing through the membrane. Next, liposomes were concentrated to 2 mL and dialyzed against 10-15 volumes of IX PBS using TFF before recovering the final product. The TFF system and hollow fiber filtration membranes were purchased from Spectrum Labs and were used according to the manufacturer's guidelines. Polysulfone hollow fiber filtration membranes with a 100 kD pore size cutoff and 8 cm² surface area were used. For *in vitro* and *in vivo* experiments, formulations were diluted to the required RNA concentration with 1X PBS.
(B) As method (A) except that, after rocking, 226.7 µL of the stock was added to 1.773 mL ethanol to make a working lipid stock solution of 2 mL, thus modifying the lipid:RNA ratio.
(C) As method (B) except that the Mustang filtration was omitted, so liposomes went from the 20 mL glass vial into the TFF dialysis.
(D) As method (C) except that the TFF used polyethersulfone (PES) hollow fiber membranes (part number P-C1-100E-100-01N) with a 100 kD pore size cutoff and 20 cm² surface area.
(E) As method (D) except that a Mustang membrane was used, as in method (A).
(F) As method (A) except that the Mustang filtration was omitted, so liposomes went from the 20 mL glass vial into the TFF dialysis.
(G) As method (D) except that a 4 mL working solution of RNA was prepared from a stock solution of ∼ 1µg/µL in 100 mM citrate buffer (pH 6). Then four 20 mL glass vials were prepared in the same way. Two of them were used for the RNA working solution (2 mL in each vial) and the others for collecting the lipid and RNA mixes, as in (C). Rather than use T mixer, syringes containing RNA and the lipids were connected to a Mitos Droplet junction Chip (a glass microfluidic device obtained from Syrris, Part no. 3000158) using PTFE tubing (0.03 inches internal diameter x 1/16 inch outer diameter) using a 4-way edge connector (Syrris). Two RNA streams and one lipid stream were driven by syringe pumps and the mixing of the ethanol and aqueous phase was done at the X junction (100 µm x 105 µm) of the chip. The flow rate of all three streams was kept at 1.5 mL/min, hence the ratio of total aqueous to ethanolic flow rate was 2:1. The tube outlet was positioned to collect the mixtures in a 20 mL glass vial (while stirring). The stir bar was taken out and the ethanol/aqueous solution was allowed to equilibrate to room temperature for 1 h. Then the mixture was loaded in a 5 cc syringe, which was fitted to another piece of the PTFE tubing; in another 5 cc syringe with equal length of PTFE tubing, an equal volume of 100 mM citrate buffer (pH 6) was loaded. The two syringes were driven at 3mL/min flow rate using a syringe pump and the final mixture collected in a 20 mL glass vial (while stirring). Next, liposomes were concentrated to 2 mL and dialyzed against 10-15 volumes of IX PBS using TFF, as in (D).
(H) As method (A) except that the 2mL working lipid stock solution was made by mixing 120.9 µL of the lipid stock with 1.879 mL ethanol. Also, after mixing in the T mixer the liposomes from the 20mL vial were loaded into Pierce Slide-A-Lyzer Dialysis Cassette (Thermo Scientific, extra strength, 0.5-3 mL capacity) and dialyzed against 400-500 mL of IX PBS overnight at 4°C in an autoclaved plastic container before recovering the final product.

After liposome formation, the percentage of encapsulated RNA and RNA concentration can be determined by Quant-iT RiboGreen RNA reagent kit (Invitrogen), following manufacturer's instructions, using the ribosomal RNA standard provided in the kit to generate a standard curve. For instance, liposomes are diluted 10x or 100x in IX TE buffer (from kit) before addition of the dye. Separately, liposomes are diluted 10x or 100x in IX TE buffer containing 0.5% Triton X before addition of the dye (to disrupt the liposomes and thus to assay total RNA). Thereafter an equal amount of dye is added to each solution and then ∼180 µL of each solution after dye addition was loaded in duplicate into a 96 well tissue culture plate. The fluorescence (Ex 485 nm, Em 528 nm) is read on a microplate reader. Liposome formulations can dosed *in vivo* based on the encapsulated amount of RNA.

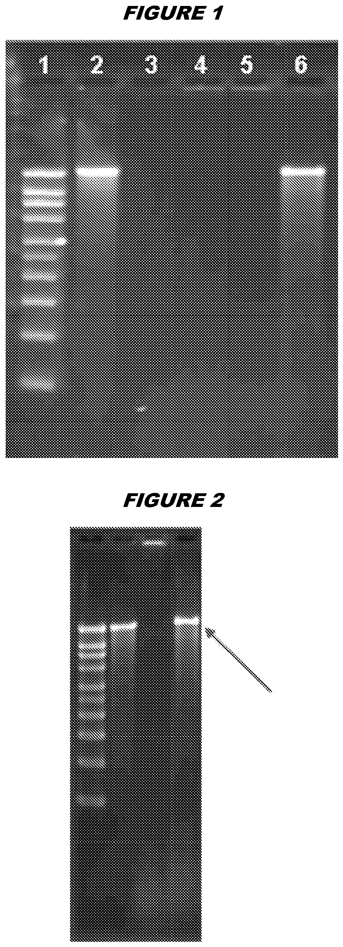

Encapsulation in liposomes was shown to protect RNA from RNase digestion. Experiments used 3.8mAU of RNase A per microgram of RNA, incubated for 30 minutes at room temperature. RNase was inactivated with Proteinase K at 55°C for 10 minutes. A 1:1 v/v mixture of sample to 25:24:1 v/v/v, phenol:chloroform:isoamyl alcohol was then added to extract the RNA from the lipids into the aqueous phase. Samples were mixed by vortexing for a few seconds and then placed on a centrifuge for 15 minutes at 12k RPM. The aqueous phase (containing the RNA) was removed and used to analyze the RNA. Prior to loading (400 ng RNA per well) all the samples were incubated with formaldehyde loading dye, denatured for 10 minutes at 65°C and cooled to room temperature. Ambion Millennium markers were used to approximate the molecular weight of the RNA construct. The gel was run at 90 V. The gel was stained using 0.1% SYBR gold according to the manufacturer's guidelines in water by rocking at room temperature for 1 hour. Figure 1 shows that RNase completely digests RNA in the absence of encapsulation (lane 3). RNA is undetectable after encapsulation (lane 4), and no change is seen if these liposomes are treated with RNase (lane 4). After RNase-treated liposomes are subjected to phenol extraction, undigested RNA is seen (lane 6). Even after 1 week at 4°C the RNA could be seen without any fragmentation (Figure 2, arrow). Protein expression *in vivo* was unchanged after 6 weeks at 4 °C and one freeze-thaw cycle. Thus liposome-encapsulated RNA is stable.

### Expression of reporter gene

To assess *in vivo* expression of RNA, a reporter enzyme (SEAP; secreted alkaline phosphatase) was encoded in the replicon, rather than an immunogen. Expression levels were measured in sera diluted 1:4 in IX Phospha-Light dilution buffer using a chemiluminescent alkaline phosphate substrate. 8-10 week old BALB/c mice (5/group) were injected intramuscularly on day 0, 50µl per leg with 0.1µg or 1µg RNA dose. The same vector was also administered without the liposomes (in RNase free IX PBS) at 1µg. Virion-packaged replicons were also tested. Virion-packaged replicons used herein (referred to as "VRPs") were obtained by the methods of reference 43, where the alphavirus replicon is derived from the mutant VEEV or a chimera derived from the genome of VEEV engineered to contain the 3' UTR of Sindbis virus and a Sindbis virus packaging signal (PS), packaged by co-electroporating them into BHK cells with defective helper RNAs encoding the Sindbis virus capsid and glycoprotein genes.

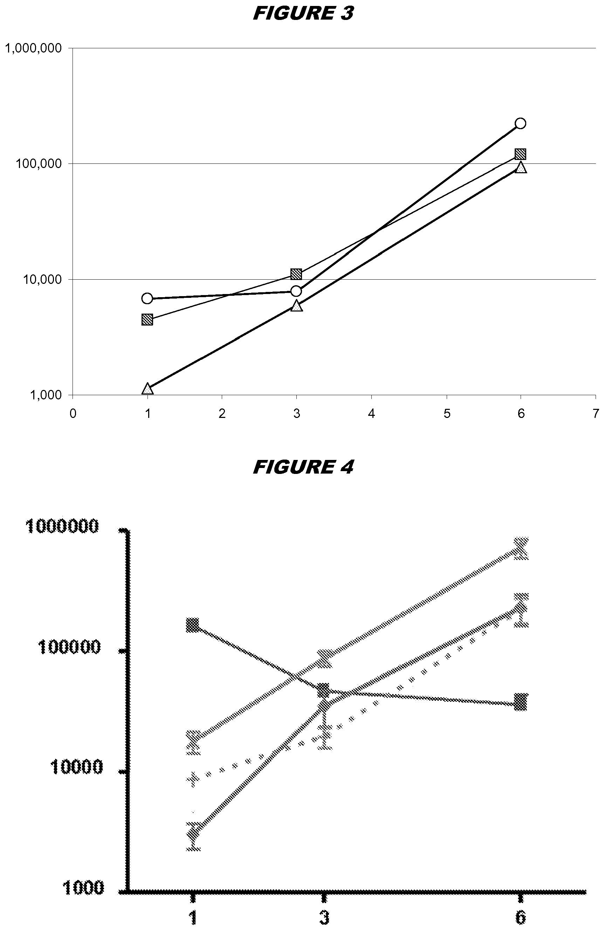

As shown in Figure 4, encapsulation increased SEAP levels by about ½ log at the 1µg dose, and at day 6 expression from a 0.1µg encapsulated dose matched levels seen with 1µg unencapsulated dose. By day 3 expression levels exceeded those achieved with VRPs (squares). Thus SEAP expression increased when the RNA was formulated in the liposomes relative to the naked RNA control, even at a 10x lower dose. Expression was also higher relative to the VRP control, but the kinetics of expression were very different (see Figure 4). Delivery of the RNA with electroporation resulted in increased expression relative to the naked control, but the levels were lower than with liposomes.

To assess whether the effect seen in the liposome groups was due merely to the liposome components, or was linked to the encapsulation, the replicon was administered in encapsulated form (with two different purification protocols, 0.1µg RNA), or mixed with the liposomes after their formation (a non-encapsulated "lipoplex", 0.1µg RNA), or as naked RNA (1µg). The lipoplex gave the lowest levels of expression, showing that shows encapsulation is essential for potent expression.

Further SEAP experiments showed a clear dose response *in vivo,* with expression seen after delivery of as little as 1ng RNA. Further experiments comparing expression from encapsulated and naked replicons indicated that 0.01µg encapsulated RNA was equivalent to 1µg of naked RNA. At a 0.5µg dose of RNA the encapsulated material gave a 12-fold higher expression at day 6; at a 0.1µg dose levels were 24-fold higher at day 6.

Rather than looking at average levels in the group, individual animals were also studied. Whereas several animals were non-responders to naked replicons, encapsulation eliminated non-responders.

### In vivo expression of immunogens

To assess *in vivo* immunogenicity a replicon was constructed to express full-length F protein from respiratory syncytial virus (RSV). This was delivered naked (1µg), encapsulated in liposomes (0.1 or 1µg), or packaged in virions (10⁶ IU; "VRP") at days 0 and 21. The liposomes clearly enhanced immunogenicity, and the RNA elicits a robust CD8 T cell response. Further experiments compared F-specific IgG titers in mice receiving VRP, 0.1µg liposome-encapsulated RNA, or 1µg liposome-encapsulated RNA. The liposome-encapsulated RNA induces essentially the same magnitude of immune response as seen with virion delivery.

A further study confirmed that the 0.1µg of liposome-encapsulated RNA gave much higher anti-F IgG responses (15 days post-second dose) than 0.1µg of delivered DNA, and even was more immunogenic than 20µg plasmid DNA encoding the F antigen, delivered by electroporation.

For studying RSV F-protein immunogenicity a self-replicating replicon "vA317" was prepared which encodes RSV F protein. This was administered to BALB/c mice, 4 or 8 animals per group, by bilateral intramuscular vaccinations (50 µL per leg) on days 0 and 21 with 1µg replicon alone or formulated as liposomes prepared with DLinDMA as described above. The PEG-DMG in these lipids included PEG-2000. For comparison, naked plasmid DNA (20 µg) expressing the same RSV-F antigen was delivered either using electroporation or with the liposomes (0.1µg DNA). Four mice were used as a naive control group. Serum was collected for antibody analysis on days 14 and 36. Spleens were harvested from mice at day 49 for T cell analysis.

F-specific serum IgG titers (GMT) were as follows, showing data for 4 different RNA-containing liposome preparations and, for comparison, the DNA-containing liposomes:

| **RV** | **Day 14** | **Day 36** |
|---|---|---|
| Naked DNA plasmid | 439 | 6712 |
| Naked A317 RNA | 78 | 2291 |
| Liposome #1 | 3020 | 26170 |
| Liposome #2 | 2326 | 9720 |
| Liposome #3 | 5352 | 54907 |
| Liposome #4 | 4428 | 51316 |
| Liposome #5 (DNA) | 5 | 13 |

Thus the liposome formulations significantly enhanced immunogenicity relative to the naked RNA controls, as determined by increased F-specific IgG titers (and also T cell frequencies; data not shows). Plasmid DNA formulated with liposomes, or delivered naked using electroporation, was significantly less immunogenic than liposome-formulated self-replicating RNA.

### Longer PEG length

To compare the effect of PEG length on *in vivo* immunogenicity, the two different sets of liposomes were prepared using method (H), either with 150µg RNA or without RNA (to make empty liposomes). Two different lipid mixtures were used, both having 40% DlinDMA, 10% DSPC, 48% cholesterol, and 2% PEG-DMG, but the two compositions used either PEG 2000 or PEG 5000. The RNA replicon was vA375 encoding the surface fusion glycoprotein of RSV.

The following table shows the size of the liposomes (Z average and polydispersity index) and the % of RNA encapsulation in each:

| **Composition** | **PEG** | **Zav (nm)** | **pdI** | **RNA** | **Encapsulation** |
|---|---|---|---|---|---|
| A | 2000 | 152.1 | 0.053 | + | 92.5% |
| B | 2000 | 144 | 0.13 | - | - |
| C | 5000 | 134 | 0.136 | + | 71.6% |
| D | 5000 | 130.3 | 0.178. | - | - |

The liposomes were administered to BALB/c mice (10 per group) by bilateral intramuscular injection (50µl per leg) on days 0 & 21. Doses were 0.01, 0.03, 0.1, 0.3 or 1µg. F-specific serum IgG and PRNT60 titers (GMT) were as follows, 2 weeks after the first or second injection:

| **Liposome** | **RNA (µg)** | **2wp1** | **2wp2** | **PRNT60 (2wp2)** |
|---|---|---|---|---|
| Buffer control | 0 | - | - | 10 |
| B | 0 | - | - | 10 |
| D | 0 | - | - | 10 |
| A | 0.01 | 3399 | 50691 | 37 |
| C | 0.01 | 3959 | 37025 | 51 |
| A | 0.03 | 3446 | 53463 | 83 |
| C | 0.03 | 5842 | 50763 | 180 |
| A | 0.1 | 8262 | 76808 | 238 |
| C | 0.1 | 7559 | 122555 | 314 |
| A | 0.3 | 5913 | 82599 | 512 |
| C | 0.3 | 5712 | 126619 | 689 |
| A | 1 | 8213 | 85138 | 441 |
| C | 1 | 9434 | 199991 | 1055 |

Inclusion of PEG 5000 elicits higher F-specific titers than the PEG 2000 after two doses of 0.1 (1.6x), 0.3 (1.5x) or 1µg (2.4x) RNA. Statistical analysis (T-test) showed that F-specific titers (2wp2) were statistically different (P<0.05) between the PEG 5000 and PEG 2000 groups at the 0.01, 0.1, 0.3 and 1 µg RNA doses. PEG 5000 gave higher neutralizing titers (2.4x) at 1 µg RNA, P<0.05.

Similar comparative experiments were performed with the vA317 replicon. Liposomes were made by method (H) with 40% DlinDMA, 10% DSPC, 48% cholesterol and 2% PEG DMG (either PEG 2000 or PEG 5000). Their characteristics were as follows:

| **Name** | **PEG** | **Zav (nm)** | **pdI** | **Encapsulation** |
|---|---|---|---|---|
| 2k | 2000 | 122.3 | 0.068 | 95.23% |
| 5k | 5000 | 106.1 | 0.136 | 61.61% |

BALB/c mice, 8 per group, were given bilateral intramuscular vaccinations (50 µL per leg) on days 0 and 21 with naked (1 µg) or liposome-encapsulated (0.1 µg) RNA. Serum was collected on days 14 and 35, and spleens were harvested on day 49.

F-specific serum IgG (GMT) were as follows, 2 weeks after the first or second injection:

| **Group** | **Day 14** | **Day 35** |
|---|---|---|
| Naked RNA | 28 | 721 |
| 2k | 2237 | 12407 |
| 5k | 5654 | 39927 |

Average net F-specific cytokine-positive T cell frequencies (CD4+ or CD8+) were as follows, showing only figures which were statistically significantly above zero (specific for RSV peptides F51-66, F164-178, F309-323 for CD4+, or for peptides F85-93 and F249-258 for CD8+):

| **Group** | **CD4-CD8+** | | | | **CD4-CD8+** | | | |
|---|---|---|---|---|---|---|---|---|
| | **IFNγ** | **IL2** | **IL5** | **TNFα** | **IFNγ** | **IL2** | **IL5** | **TNFα** |
| Naked | 0.02 | 0.02 | 0.04 | | 0.36 | 0.16 | | 0.28 |
| 2k | 0.03 | 0.04 | | 0.03 | 0.66 | 0.17 | | 0.56 |
| 5k | 0.06 | 0.08 | | 0.07 | 1.42 | 0.46 | | 1.09 |

Thus F-specific IgG titers were increased 2.5-fold (2wp1) and 3-fold (2wp2) by increasing the molecular weight of the PEG head group from 2000 to 5000. There was also a positive impact on T cell responses.

### Effect of PEG length up to 10kDa

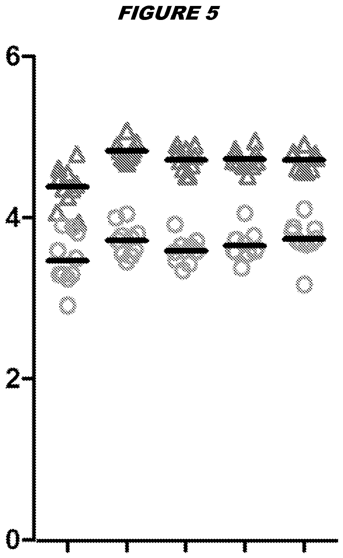

BALB/c mice were used to study the impact of different PEG lengths in the range from 1-10kDa. Five groups received two doses (days 0 & 21) 0.1µg RNA encoding RSV F protein (vA375 replicon) in liposomes formed from DLinDMA cationic lipid and different lengths of PEG-DMG (1kDa, 2kDa, 3kDa, 5kDa, 10kDa). F-specific IgG titers were measured 14 days after the first and second doses and the results are shown in Figure 5. The data show that PEG lengths of 2, 3, 5 and 10 kDa provide essentially the same titers at both time points, and these are all better than seen with 1 kDa PEG.

Control animals received DLinDMA liposomes with 2kDa PEG-DMG, but no RNA. These animals showed an anti-F IgG titer which also increased after the second dose (but at much lower levels than in the animals receiving encapsulated RNA). Serum samples from this group did not show a non-specific response on gp140 or PBS-coated ELISA plates.

Serum cytokines were measured 5 hours after injections of the liposomes. In general, no IL-1β or TNF-α responses were seen in any mice, and responses for IL-12/p70, IL-6, IL-10, IFN-γ and IP-10 were equivalent for all PEG lengths. The KC/GRO response was significantly lower in mice who received liposomes with 5kDa or 10kDa PEG-DMG than in liposomes without PEGylation.

### PEG5000 studies with RSV

Four different replicons were used for this study, all encoding full-length wild type F glycoprotein of RSV with the fusion peptide deleted. The vA372 replicon is formed by runoff transcription. The 3' end of the vA142 replicon is formed by ribozyme mediated cleavage. In the vA368 expression of the protein is driven by the EV71 internal ribosome entry site (IRES). In the vA369 replicon expression is driven by the EMCV IRES.

Liposomes were formed with 40% RV17 cationic lipid, 10% DSPC, 49.5% cholesterol, 0.5% PEG DMG 5000, made using method (H) with a 175 µg RNA batch size.

BALB/c mice, 7 animals per group, were given bilateral intramuscular vaccinations (50 µL per leg) on days 0 and 21 with:
**Group 1** self-replicating RNA (vA372, 1.0 µg) formulated in liposomes
**Group 2** self-replicating RNA (vA142, 1.0 µg) formulated in liposomes
**Group 3** VRP containing the vA142 RNA (1x10⁶ IU)
**Group 4** self-replicating RNA (vA368, 1.0 µg) formulated in liposomes
**Group 5** VRP containing the vA368 RNA (1x10⁶ IU)
**Group 6** self-replicating RNA (vA369, 1.0 µg) formulated in liposomes
**Group 7** VRP containing the vA369 RNA (1x10⁶ IU)
**Group 8** Naive control (4 animals)

Sera were collected for antibody analysis on days 0, 20, 35. Spleens were harvested on day 35 for T-cell analysis.

F-specific serum IgG titers and neutralisation titers (GMT) were as follows:

| **Group** | **IgG Day 20** | **IgG Day 35** | **Neutralⁿ** |
|---|---|---|---|
| 1 | 4678 | 76715 | 195 |
| 2 | 2471 | 51963 | 116 |
| 3 | 2898 | 42441 | 202 |
| 4 | 1463 | 33194 | 134 |
| 5 | 2236 | 33456 | 65 |
| 6 | 1524 | 37330 | 49 |
| 7 | 2785 | 31640 | 66 |
| 8 | 5 | 5 | - |

Thus all four replicons were immunogenic and each elicited serum F-specific IgG antibodies after the first vaccination, with the second vaccination boosting the response effectively. RSV neutralizing antibodies were detected after the second vaccination. Similar post-second vaccination antibody titers were induced by a replicon in which 3' end was formed by ribozyme-mediated cleavage (vA142) and a replicon in which the 3' end was formed by runoff transcription (vA372). EV71 or EMCV-driven expression of the F antigen did not enhance the antibody response to the replicon (vA368 or vA369 *vs.* vA142). Similarly, T cell responses (not shown) did not differentiate replicons in which the 3' end was formed by ribozyme-mediated cleavage (vA142) or runoff transcription (vA372), and did not show a benefit to EV71 or EMCV-driven expression of the F antigen (vA238 or vA369 vs. vA142).

The vA142 replicon was also tested in cotton rats (*Sigmodon hispidis*) using liposomes formed from:
(a) 40% DlinDMA, 10% DPSC, 48% cholesterol and 2% PEG DMG 2000, made by method (D) with a 175µg RNA batch size
(b) 40% RV17, 10% DSPC, 49.5% cholesterol and 0.5% PEG DMG 5000, made using method (H) with a 200µg RNA batch size.
(c) 40% RV05, 30% DLoPE (18:2 PE), 28% cholesterol and 2% PEG DMG 2000, made using method (H) with a 200µg RNA batch size.

Cotton rats, 4-8 animals per group, were given intramuscular vaccinations (100 µL in one leg) on days 0 and 21 with:
**Group 1** self-replicating RNA (vA142, 1 µg, RSV-F) formulated in liposomes (a)
**Group 2** self-replicating RNA (vA142, 0.1 µg, RSV-F) formulated in liposomes (a)
**Group 3** self-replicating RNA (vA142, 1 µg, RSV-F) formulated in liposomes (b)
**Group 4** self-replicating RNA (vA142, 0.1 µg, RSV-F) formulated in liposomes (b)
**Group 5** self-replicating RNA (vA142, 1 µg, RSV-F) formulated in liposomes (c)
**Group 6** self-replicating RNA (vA142, 0.1 µg, RSV-F) formulated in liposomes (c)
**Group 7** VRPs (1x10⁶ IU) expressing the full-length wild type surface F glycoprotein of RSV
**Group 8** RSV-F subunit protein vaccine (5 µg) adjuvanted with aluminium hydroxide
**Group 9** a naive control (3 animals)

All cotton rats (except group 9) were vaccinated with 5 µg F subunit + aluminium hydroxide on day 49 (four weeks after the second vaccination).

Serum was collected for antibody analysis on days 0, 21, 35, 49, 64.

F-specific serum IgG titers (GMT) were as follows:

| **Group** | **Day 21** | **Day 35** | **Day 49** | **Day 64** |
|---|---|---|---|---|
| 1 | 558 | 3938 | 2383 | 16563 |
| 2 | 112 | 1403 | 943 | 15123 |
| 3 | 330 | 2927 | 2239 | 25900 |
| 4 | 51 | 503 | 503 | 20821 |
| 5 | 342 | 3207 | 2151 | 24494 |
| 6 | 49 | 1008 | 513 | 15308 |
| 7 | 1555 | 7448 | 4023 | 25777 |
| 8 | 8425 | 81297 | 54776 | 82911 |
| 9 | 5 | 5 | 5 | 5 |

RSV serum neutralizing antibody titers were as follows:

| **Group** | **Day 21** | **Day 35** | **Day 49** | **Day 64** |
|---|---|---|---|---|
| 1 | 66 | 788 | 306 | 161 |
| 2 | 26 | 162 | 58 | 1772 |
| 3 | 69 | 291 | 198 | 3221 |
| 4 | 24 | 72 | 43 | 1135 |
| 5 | 75 | 448 | 201 | 5733 |
| 6 | 27 | 371 | 163 | 2449 |
| 7 | 137 | 2879 | 1029 | 1920 |
| 8 | 307 | 2570 | 1124 | 2897 |
| 9 | 10 | - | - | 10 |

The protein vaccination did not boost antibody titers in cotton rats previously vaccinated with protein, but it provided a large boost to titers in cotton rats previously vaccinated with RNA. In most cases the RSV serum neutralization titers after two RNA vaccinations followed by protein were equal to titers induced by two or three sequential adjuvanted protein vaccinations.

### CMV immunogenicity

Liposomes were used to deliver RNA replicons encoding cytomegalovirus (CMV) glycoproteins. The "vA160" replicon encodes full-length glycoproteins H and L (gH/gL), whereas the "vA322" replicon encodes a soluble form (gHsol/gL). The two proteins are under the control of separate subgenomic promoters in a single replicon; co-administration of two separate vectors, one encoding gH and one encoding gL, did not give good results.

BALB/c mice, 10 per group, were given bilateral intramuscular vaccinations (50 µL per leg) on days 0, 21 and 42 with VRPs expressing gH/gL (1x10⁶ IU), VRPs expressing gHsol/gL (1x10⁶ IU) and PBS as the controls. Two test groups received 1µg of the vA160 or vA322 replicon formulated in liposomes (40% DlinDMA, 10% DSPC, 48% Chol, 2% PEG-DMG 2000; made using method (D) but with 150µg RNA batch size).

The vA160 liposomes had a Zav (Z-average) diameter of 168nm, a pdI (polydispersity index) of 0.144, and 87.4% encapsulation. The vA322 liposomes had a Zav diameter of 162nm, a pdI of 0.131, and 90% encapsulation.

The replicons were able to express two proteins from a single vector.

Sera were collected for immunological analysis on day 63 (3wp3). CMV neutralization titers (the reciprocal of the serum dilution producing a 50% reduction in number of positive virus foci per well, relative to controls) were as follows:

| **gH/gL VRP** | **gHsol/gL VRP** | **gH/gL liposome** | **gHsol/gL liposome** |
|---|---|---|---|
| 4576 | 2393 | 4240 | 10062 |

RNA expressing either a full-length or a soluble form of the CMV gH/gL complex thus elicited high titers of neutralizing antibodies, as assayed on epithelial cells. The average titers elicited by the liposome-encapsulated RNAs were at least as high as for the corresponding VRPs.

Repeat experiments confirmed that the replicon was able to express two proteins from a single vector. The RNA replicon gave a 3wp3 titer of 11457, compared to 5516 with VRPs.

Further experiments used different replicons in addition to vA160, and used a longer PEG in the liposomes. The vA526 replicon expresses the CMV pentameric complex (gH-gL-UL128-UL130-UL-131) under the control of three subgenomic promoters: the first drives the expression of gH; the second drives expression of gL; the third drives the expression of the UL128-2A-UL130-2A-UL131 polyprotein, which contains two 2A cleavage sites between the three UL genes. The vA527 replicon expresses the CMV pentameric complex via three subgenomic promoters and two IRESs: the first subgenomic promoter drives the expression of gH; the second subgenomic promoter drives expression of gL; the third subgenomic promoter drives the expression of the UL128; UL130 is under the control of an EMCV IRES; UL131 is under control of an EV71 IRES. These three replicons were delivered by liposome (prepared by method (H), with 150µg batch size; 40% DlinDMA, 10% DSPC, 48% cholesterol, 2% PEG DMG 5000) or by VRPs.

BALB/c mice, 10 groups of 10 animals, were given bilateral intramuscular vaccinations (50 µL per leg) on days 0, 21 and 42 with:
Group 1 VRPs expressing gH FL/gL (1x10⁶ IU)
Group 2 pentameric, 2A VRP (1x10⁵ IU)
Group 3 pentameric, 2A VRP (1x10⁶ IU)
Group 4 pentameric, IRES VRP (1x10⁵ IU)
Group 5 self-replicating RNA vA160 (1µg) formulated in liposomes
Group 6 self-replicating RNA vA526 (1µg) formulated in liposomes
Group 7 self-replicating RNA vA527 (1µg) formulated in liposomes
Group 8 self-replicating RNA vA160 (1µg) formulated in a cationic nanoemulsion
Group 9 self-replicating RNA vA526 (1µg) formulated in a cationic nanoemulsion
Group 10 self-replicating RNA vA527 (1µg) formulated in a cationic nanoemulsion.

Sera were collected for immunological analysis on days 21 (3wp1), 42 (3wp2) and 63 (3wp3). CMV serum neutralization titers on days 21, 42 and 63 were:

| **Vaccine Group** | **3wp1** | **3wp2** | **3wp3** |
|---|---|---|---|
| 1 | 126 | 6296 | 26525 |
| 2 | N/A | N/A | 6769 |
| 3 | N/A | 3442 | 7348 |
| 4 | N/A | N/A | 2265 |
| 5 | 347 | 9848 | 42319 |
| 6 | 179 | 12210 | 80000 |
| 7 | 1510 | 51200 | 130000 |
| 8 | N/A | N/A | 845 |
| 9 | N/A | N/A | 228 |
| 10 | N/A | N/A | 413 |

Thus self-replicating RNA can be used to express multiple antigens from a single vector and to raise a potent and specific immune response. The replicon can express five antigens (CMV pentameric complex (gH-gL-UL128-UL130-UL-131) and raise a potent immune response. Self-replicating RNA delivered in liposomes with PEG5000 was able to elicit high titers of neutralizing antibody, as assayed on epithelial cells, at all time points assayed (3wp1, 3wp2, and 3wp3). These responses were superior to the corresponding VRPs and to cationic nanoemulsions.

**Table 1: useful phospholipids**

| | |
|---|---|
| DDPC | 1,2-Didecanoyl-sn-Glycero-3-phosphatidylcholine |
| DEPA | 1,2-Dierucoyl-sn-Glycero-3-Phosphate |
| DEPC | 1,2-Erucoyl-sn-Glycero-3-phosphatidylcholine |
| DEPE | 1,2-Dierucoyl-sn-Glycero-3-phosphatidylethanolamine |
| DEPG | 1,2-Dierucoyl-sn-Glycero-3[Phosphatidyl-rac-(1-glycerol...) |
| DLOPC | 1,2-Linoleoyl-sn-Glycero-3-phosphatidylcholine |
| DLPA | 1,2-Dilauroyl-sn-Glycero-3-Phosphate |
| DLPC | 1,2-Dilauroyl-sn-Glycero-3-phosphatidylcholine |
| DLPE | 1,2-Dilauroyl-sn-Glycero-3-phosphatidylethanolamine |
| DLPG | 1,2-Dilauroyl-sn-Glycero-3[Phosphatidyl-rac-(1-glycerol...) |
| DLPS | 1,2-Dilauroyl-sn-Glycero-3-phosphatidylserine |
| DMG | 1,2-Dimyristoyl-sn-glycero-3-phosphoethanolamine |
| DMPA | 1,2-Dimyristoyl-sn-Glycero-3-Phosphate |
| DMPC | 1,2-Dimyristoyl-sn-Glycero-3-phosphatidylcholine |
| DMPE | 1,2-Dimyristoyl-sn-Glycero-3-phosphatidylethanolamine |
| DMPG | 1,2-Myristoyl-sn-Glycero-3[Phosphatidyl-rac-(1-glycerol...) |
| DMPS | 1,2-Dimyristoyl-sn-Glycero-3-phosphatidylserine |
| DOPA | 1,2-Dioleoyl-sn-Glycero-3-Phosphate |
| DOPC | 1,2-Dioleoyl-sn-Glycero-3-phosphatidylcholine |
| DOPE | 1,2-Dioleoyl-sn-Glycero-3-phosphatidylethanolamine |
| DOPG | 1,2-Dioleoyl-sn-Glycero-3[Phosphatidyl-rac-(1-glycerol...) |
| DOPS | 1,2-Dioleoyl-sn-Glycero-3-phosphatidylserine |
| DPPA | 1,2-Dipalmitoyl-sn-Glycero-3-Phosphate |
| DPPC | 1,2-Dipalmitoyl-sn-Glycero-3-phosphatidylcholine |
| DPPE | 1,2-Dipalmitoyl-sn-Glycero-3-phosphatidylethanolamine |
| DPPG | 1,2-Dipalmitoyl-sn-Glycero-3[Phosphatidyl-rac-(1-glycerol...) |
| DPPS | 1,2-Dipalmitoyl-sn-Glycero-3-phosphatidylserine |
| DPyPE | 1,2-diphytanoyl-sn-glycero-3-phosphoethanolamine |
| DSPA | 1,2-Distearoyl-sn-Glycero-3-Phosphate |
| DSPC | 1,2-Distearoyl-sn-Glycero-3-phosphatidylcholine |
| DSPE | 1,2-Diostearpyl-sn-Glycero-3-phosphatidylethanolamine |
| DSPG | 1,2-Distearoyl-sn-Glycero-3[Phosphatidyl-rac-(1-glycerol...) |
| DSPS | 1,2-Distearoyl-sn-Glycero-3-phosphatidylserine |
| EPC | Egg-PC |
| HEPC | Hydrogenated Egg PC |
| HSPC | High purity Hydrogenated Soy PC |
| HSPC | Hydrogenated Soy PC |
| LYSOPC MYRISTIC | 1-Myristoyl-sn-Glycero-3-phosphatidylcholine |
| LYSOPC PALMITIC | 1-Palmitoyl-sn-Glycero-3-phosphatidylcholine |
| LYSOPCSTEARIC | 1-Stearoyl-sn-Glycero-3-phosphatidylcholine |
| Milk Sphingomyelin MPPC | 1-Myristoyl,2-palmitoyl-sn-Glycero 3-phosphatidylcholine |
| MSPC | 1-Myristoyl,2-stearoyl-sn-Glycero-3-phosphatidylcholine |
| PMPC | 1-Palmitoyl,2-myristoyl-sn-Glycero-3-phosphatidylcholine |
| POPC | 1-Palmitoyl,2-oleoyl-sn-Glycero-3-phosphatidylcholine |
| POPE | 1-Palmitoyl-2-oleoyl-sn-Glycero-3-phosphatidylethanolamine |
| POPG | 1,2-Dioleoyl-sn-Glycero-3[Phosphatidyl-rac-(1-glycerol)...] |
| PSPC | 1-Palmitoyl,2-stearoyl-sn-Glycero-3-phosphatidylcholine |
| SMPC | 1-Stearoyl,2-myristoyl-sn-Glycero-3-phosphatidylcholine |
| SOPC | 1-Stearoyl,2-oleoyl-sn-Glycero-3-phosphatidylcholine |
| SPPC | 1-Stearoyl,2-palmitoyl-sn-Glycero-3-phosphatidylcholine |

### REFERENCES

[1] Johanning et al. (1995) Nucleic Acids Res 23:1495-1501.
[2] WO2011/057020.
[3] WO2011/076807.
[4] Heyes et al. (2005) J Controlled Release 107:276-87.
[5] WO2005/121348.
*[6]* Liposomes: Methods and Protocols, Volume 1: Pharmaceutical Nanocarriers: Methods and Protocols, (ed. Weissig). Humana Press, 2009. ISBN 160327359X.
*[7]* Liposome Technology, volumes I, II & III. (ed. Gregoriadis). Informa Healthcare, 2006.
*[8]* Functional Polymer Colloids and Microparticles volume 4 (Microspheres, microcapsules & liposomes). (eds. Arshady & Guyot). Citus Books, 2002.
[9] Jeffs et al. (2005) Pharmaceutical Research 22 (3):362-372.
[10] WO2005/113782.
[11] WO2011/005799.
[12] El Ouahabi et al. (1996) FEBS Letts 380:108-12.
[13] Giuliani et al. (2006) Proc Natl Acad Sci U S A 103(29):10834-9.
[14] WO2009/016515.
[15] WO02/34771.
[16] WO2005/032582.
[17] WO2010/119343.
[18] WO2006/110413.
[19] WO2005/111066.
[20] WO2005/002619.
[21] WO2006/138004.
[22] WO2009/109860.
[23] WO02/02606.
[24] WO03/018054.
[25] WO2006/091517.
[26] WO2008/020330.
[27] WO2006/089264.
[28] WO2009/104092.
[29] WO2009/031043.
[30] WO2007/049155.
[31] Gennaro (2000) Remington: The Science and Practice of Pharmacy. 20th edition, ISBN: 0683306472.
[32] Romberg et al. (2008) Pharmaceutical Research 25:55-71.
[33] Hoekstra et al., Biochimica et Biophysica Acta 1660 (2004) 41-52
[34] Methods In Enzymology (S. Colowick and N. Kaplan, eds., Academic Press, Inc.)
[35] Handbook of Experimental Immunology, Vols. I-IV (D.M. Weir and C.C. Blackwell, eds, 1986, Blackwell Scientific Publications)
[36] Sambrook et al. (2001) Molecular Cloning: A Laboratory Manual, 3rd edition (Cold Spring Harbor Laboratory Press).
[37] Handbook of Surface and Colloidal Chemistry (Birdi, K.S. ed., CRC Press, 1997)
[38] Ausubel et al. (eds) (2002) Short protocols in molecular biology, 5th edition (Current Protocols).
[39] Molecular Biology Techniques: An Intensive Laboratory Course, (Ream et al., eds., 1998)
[40] PCR (Introduction to Biotechniques Series), 2nd ed. (Newton & Graham eds., 1997)
[41] Yoneyama & Fujita (2007) Cytokine & Growth Factor Reviews 18:545-51.
[42] Maurer et al. (2001) Biophysical Journal, 80: 2310-2326.
[43] Perri et al. (2003) J Virol 77:10394-10403.

## Claims

1. A liposome within which RNA encoding an immunogen of interest is encapsulated, wherein the liposome comprises at least one lipid that is modified by covalent attachment of a polyethylene glycol moiety, such that polyethylene glycol is present on the liposome's exterior, wherein the average molecular mass of the polyethylene glycol is above 3kDa but less than 11kDa, and wherein the liposome does not contain ribosomes.

2. The liposome of claim 1, comprising PEG-DMG and/or
(i) a lipid of formula (X): 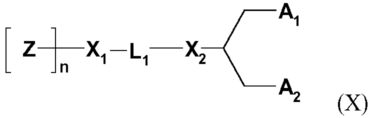 wherein:
[Z]ₙ is a hydrophilic PEG head group component polymerized by n subunits, wherein n is a number-averaged degree of polymerization between 10 and 200 units of Z, and wherein the PEG may be linear or branched, and may be optionally substituted;
L₁ is an optionally substituted C₁₋₁₀ alkylene or C₁₋₁₀ heteroalkylene linker including zero, one or two of an ether (e.g., -O-), ester (e.g., -C(O)O-), succinate (e.g., -O(O)C-CH₂-CH₂-C(O)O-)), carbamate (e.g., -OC(O)-NR'-), carbonate (e.g., -OC(O)O-), urea (e.g., -NRC(O)NR'-), amine (e.g., -NR'-), amide (e.g., -C(O)NR'-), imine (e.g., -C(NR')-), thioether (e.g., -S-), xanthate (e.g., -OC(S)S-), and phosphodiester (e.g., -OP(O)₂O-), wherein R' is independently selected from -H, -NH-, -NH₂, -O-, -S-, a phosphate or an optionally substituted C₁₋₁₀ alkylene;
X₁ and X₂ are independently selected from a carbon or a heteroatom selected from - NH-, -O-, -S- or a phosphate;
A₁ and A₂ are either independently selected from a C₆₋₃₀ alkyl, C₆₋₃₀ alkenyl, and C₆₋₃₀ alkynyl, wherein A₁ and A₂ may be the same or different, or A₁ and A₂ together with the carbon atom to which they are attached form an optionally substituted steroid; and/or
(ii) a lipid of formula (X'): 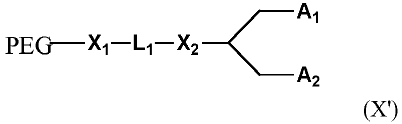 wherein:
PEG is a poly(ethylene glycol) polymerized by n subunits, wherein n is a number-averaged degree of polymerization between 70 and 240 units of ethylene oxide, wherein the PEG may be linear or branched, and may be optionally substituted;
L₁ is an optionally substituted C₁₋₁₀ heteroalkylene linker containing one or two of an ether, ester, succinate, carbamate, carbonate, urea, amine, amide, imine, thioether, xanthate, and phosphodiester;
X₁ and X₂ are oxygen;
A₁ and A₂ are independently selected from a C₆₋₃₀ alkyl, C₆₋₃₀ alkenyl, and C₆₋₃₀ alkynyl, wherein A₁ and A₂ may be the same or different, or wherein A₁ and A₂ together with the carbon atom to which they are attached form an optionally substituted steroid

3. The liposome of claim 1, wherein the lipid that is modified by covalent attachment of a polyethylene glycol moiety comprises the PEG structure: 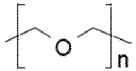 wherein n is between 70 and 240, for example wherein n is about 113, and optionally wherein the PEG moiety terminates with an -O methyl group.

4. The liposome of claim 3, wherein the lipid comprises the structure:

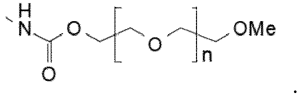

5. The liposome of any preceding claim, wherein the liposome has a diameter in the range of 80-160nm.

6. The liposome of any preceding claim, wherein the liposome comprises a lipid with a cationic head group.

7. The liposome of any preceding claim, wherein the liposome comprises a lipid with a zwitterionic head group.

8. The liposome of any preceding claim, wherein the RNA is a self-replicating RNA molecule.

9. The liposome of claim 8, wherein the self-replicating RNA molecule encodes (i) a RNA dependent RNA polymerase which can transcribe RNA from the self-replicating RNA molecule and (ii) an immunogen.

10. The liposome of claim 9, wherein the self-replicating RNA molecule has two open reading frames, the first of which encodes an alphavirus replicase and the second of which encodes the immunogen.

11. The liposome of any preceding claim, wherein the RNA molecule is 9000-12000 nucleotides long.

12. The liposome of any preceding claim, wherein the immunogen can elicit an immune response *in vivo* against a bacterium, a virus, a fungus or a parasite.

13. A pharmaceutical composition comprising a liposome of any preceding claim.

14. The liposome of any one of claims 1-12, or the pharmaceutical composition of claim 13, for use in raising a protective immune response in a vertebrate.

## Patentansprüche

1. Liposom, innerhalb dessen RNA, die ein interessierendes Immunogen codiert, eingekapselt ist, wobei das Liposom wenigstens ein Lipid umfasst, welches durch kovalentes Anheften einer Polyethylenglycolgruppe modifiziert ist, so dass Polyethylenglycol an der Außenseite des Liposoms vorliegt, wobei die durchschnittliche molekulare Masse des Polyethylenglycols oberhalb von 3 kDa, jedoch weniger als 11 kDa ist, und wobei das Liposom keine Ribosome enthält.

2. Liposom gemäß Anspruch 1, umfassend PEG-DMG und/oder
(i) ein Lipid der Formel (X): 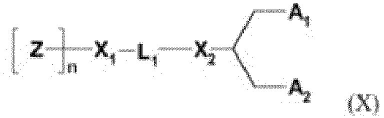 wobei:
[Z]ₙ eine hydrophile PEG-Kopfgruppenkomponente, polymerisiert durch n Untereinheiten, ist, wobei n ein auf das Zahlenmittel bezogener Polymerisationsgrad zwischen 10 und 200 Einheiten von Z ist und wobei das PEG linear oder verzweigt sein kann und optional substituiert sein kann;
L₁ ein optional substituierter C₁₋₁₀-Alkylen- oder C₁₋₁₀-Heteroalkylen-Linker einschließlich Null ist, ein oder zwei von einem Ether (z. B. -0-), Ester (z. B. -C(O)O-), Succinat (z. B. -O(O)C-CH₂-CH₂-C(O)O-)), Carbamat (z. B. -OC(O)-NR'-), Carbonat (z. B. -OC(O)O-), Harnstoff (z. B. -NRC(O)NR'-), Amin (z. B. -NR'-), Amid (z. B. -C(O)NR'-), Imin (z. B. -C(NR')-), Thioether (z. B. -S-), Xanthat (z. B. -OC(S)S-) und Phosphodiester (z. B. - OP(O)₂O-), wobei R' unabhängig aus -H, -NH-, -NH₂, -0-, -S-, einem Phosphat oder einem optional substituierten C₁₋₁₀-Alkylen ausgewählt ist;
X₁ und X₂ unabhängig aus einem Kohlenstoff oder einem Heteroatom, ausgewählt aus -NH-, -0-, -S- oder einem Phosphat, ausgewählt sind;
A₁ und A₂ entweder unabhängig aus einem C₆₋₃₀-Alkyl, C₆₋₃₀-Alkenyl und C₆₋₃₀-Alkynyl ausgewählt sind, wobei A₁ und A₂ die gleichen oder unterschiedlich sein können oder A₁ und A₂ zusammen mit dem Kohlenstoffatom, an das sie angeheftet sind, ein optional substituiertes Steroid bilden; und/oder
(ii) ein Lipid der Formel (X'): 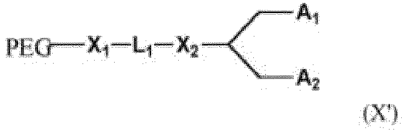 wobei:
PEG ein Poly(ethylenglycol), polymerisiert durch n Untereinheiten, ist, wobei n ein auf das Zahlenmittel bezogener Polymerisationsgrad zwischen 70 und 240 Einheiten von Ethylenoxid ist, wobei das PEG linear oder verzweigt sein kann und optional substituiert sein kann;
L₁ ein optional substituierter C₁₋₁₀-Heteroalkylen-Linker ist, der ein oder zwei von einem Ether, Ester, Succinat, Carbamat, Carbonat, Harnstoff, Amin, Amid, Imin, Thioether, Xanthat und Phosphodiester enthält;
X₁ und X₂ Sauerstoff sind;
A₁ und A₂ unabhängig aus einem C₆₋₃₀-Alkyl, C₆₋₃₀-Alkenyl und C₆₋₃₀-Alkynyl ausgewählt sind, wobei A₁ und A₂ die gleichen oder unterschiedlich sein können oder wobei A₁ und A₂ zusammen mit dem Kohlenstoffatom, an das sie angeheftet sind, ein optional substituiertes Steroid bilden.

3. Liposom gemäß Anspruch 1, wobei das Lipid, das durch kovalentes Anheften einer Polyethylenglycolgruppe modifiziert ist, die PEG-Struktur umfasst: 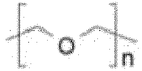 wobei n zwischen 70 und 240 ist, zum Beispiel wobei n etwa 113 ist, und wobei optional die PEG-Gruppe mit einer -O-Methylgruppe endet.

4. Liposom gemäß Anspruch 3, wobei das Lipid die Struktur umfasst:

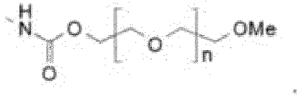

5. Liposom gemäß irgendeinem vorangegangenen Anspruch, wobei das Liposom einen Durchmesser im Bereich von 80-160 nm hat.

6. Liposom gemäß irgendeinem vorangegangenen Anspruch, wobei das Liposom ein Lipid mit einer kationischen Kopfgruppe umfasst.

7. Liposom gemäß irgendeinem vorangegangenen Anspruch, wobei das Liposom ein Lipid mit einer zwitterionischen Kopfgruppe umfasst.

8. Liposom gemäß irgendeinem vorangegangenen Anspruch, wobei die RNA ein selbstreplizierendes RNA-Molekül ist.

9. Liposom gemäß Anspruch 8, wobei das selbstreplizierende RNA-Molekül (i) eine RNA-abhängige RNA-Polymerase, die RNA aus dem selbstreplizierenden RNA-Molekül transkribieren kann, und (ii) ein Immunogen codiert.

10. Liposom gemäß Anspruch 9, wobei das selbstreplizierende RNA-Molekül zwei offene Leserahmen hat, von denen der erste eine Alphavirus-Replikase codiert und von denen der zweite das Immunogen codiert.

11. Liposom gemäß irgendeinem vorangegangenen Anspruch, wobei das RNA-Molekül 9000-12000 Nukleotide lang ist.

12. Liposom gemäß irgendeinem vorangegangenen Anspruch, wobei das Immunogen eine Immunantwort *in vivo* gegen ein Bakterium, ein Virus, einen Pilz oder einen Parasiten auslösen kann.

13. Pharmazeutische Zusammensetzung, welche ein Liposom gemäß irgendeinem vorangegangenen Anspruch umfasst.

14. Liposom gemäß irgendeinem der Ansprüche 1-12 oder pharmazeutische Zusammensetzung gemäß Anspruch 13 zur Verwendung beim Hervorrufen einer schützenden Immunantwort in einem Wirbeltier.

## Revendications

1. Liposome dans lequel l'ARN codant pour un immunogène d'intérêt est encapsulé, dans lequel le liposome comprend au moins un lipide qui est modifié par fixation covalente d'un fragment de polyéthylène glycol, de telle sorte que le polyéthylène glycol soit présent à l'extérieur du liposome, dans lequel la masse moléculaire moyenne du polyéthylène glycol est supérieure à 3 kDa mais inférieure à 11 kDa, et dans lequel le liposome ne contient pas de ribosomes.

2. Liposome selon la revendication 1, comprenant du PEG-DMG et/ou
(i) un lipide de formule (X) : 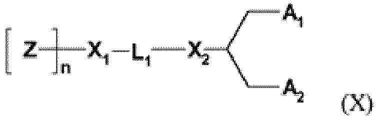 dans lequel :
[Z]ₙ est un composant de groupe de tête de PEG hydrophile polymérisé par n sous-unités, dans lequel n est un degré de polymérisation moyen en nombre compris entre 10 et 200 unités de Z, et le PEG pouvant être linéaire ou ramifié, et peut être facultativement substitué ;
L₁ est un lieur alkylène en C₁₋₁₀ ou hétéroalkylène en C₁₋₁₀ facultativement substitué, comprenant zéro, un ou deux d'un éther (par exemple, -O-), un ester (par exemple, -C(O)O-), un succinate (par exemple, -O(O)C-CH₂-CH₂-C(O)O-)), un carbamate (par exemple -OC(O)-NR'-), un carbonate (par exemple -OC(O)O-), une urée (par exemple - NRC(O)NR'-), une amine (par exemple, -NR'-), un amide (par exemple, - C(O)NR'-), une imine (par exemple, -C(NR')-), un thioéther (par exemple, - S-), un xanthate (par exemple, -OC(S)S-) et un phosphodiester (par exemple, -OP(O)₂O-), dans lequel R' est indépendamment choisi parmi -H, -NH-, -NH₂-, -O-, -S-, un phosphate ou un alkylène en C₁₋₁₀ facultativement substitué ;
X₁ et X₂ sont choisis indépendamment parmi un carbone ou un hétéroatome choisi parmi -NH-, -O-, -S- ou un phosphate ;
A₁ et A₂ sont choisis indépendamment parmi un alkyle en C₆₋₃₀, un alcényle en C₆₋₃₀ et un alcynyle en C₆₋₃₀, dans lequel A₁ et A₂ peuvent être identiques ou différents, ou A₁ et A₂ conjointement avec l'atome de carbone auquel ils sont liés forment un stéroïde facultativement substitué ; et/ou
(ii) un lipide de formule (X') : 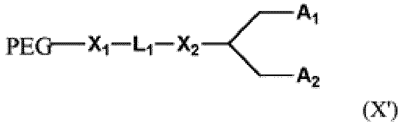 dans lequel :
le PEG est un poly(éthylène glycol) polymérisé par n sous-unités, dans lequel n est un degré de polymérisation moyen en nombre compris entre 70 et 240 unités d'oxyde d'éthylène, le PEG pouvant être linéaire ou ramifié, et pouvant être facultativement substitué ;
L₁ est un lieur hétéroalkylène en C₁₋₁₀ facultativement substitué contenant un ou deux parmi un éther, un ester, un succinate, un carbamate, un carbonate, une urée, une amine, un amide, une imine, un thioéther, un xanthate et un phosphodiester ;
X₁ et X₂ sont de l'oxygène ;
A₁ et A₂ sont indépendamment choisis parmi un alkyle en C₆₋₃₀, un alcényle en C₆₋₃₀ et un alcynyle en C₆₋₃₀, dans lequel A₁ et A₂ peuvent être identiques ou différents, ou dans lequel A₁ et A₂ forment avec l'atome de carbone auquel ils sont liés un stéroïde facultativement substitué.

3. Liposome selon la revendication 1, dans lequel le lipide modifié par fixation covalente d'un fragment de polyéthylène glycol comprend la structure PEG : 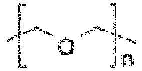 dans lequel n est compris entre 70 et 240, par exemple dans lequel n est environ 113, et facultativement dans lequel le fragment PEG se termine par un groupe -O méthyle.

4. Liposome selon la revendication 3, dans lequel le lipide comprend la structure :

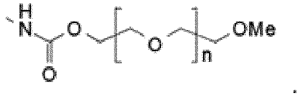

5. Liposome selon l'une quelconque des revendications précédentes, dans lequel le liposome a un diamètre compris entre 80 et 160 nm.

6. Liposome selon l'une quelconque des revendications précédentes, dans lequel le liposome comprend un lipide avec un groupe de tête cationique.

7. Liposome selon l'une quelconque des revendications précédentes, dans lequel le liposome comprend un lipide avec un groupe de tête zwitterionique.

8. Liposome selon l'une quelconque des revendications précédentes, dans lequel l'ARN est une molécule d'ARN auto-répliquant.

9. Liposome selon la revendication 8, dans lequel la molécule d'ARN auto-répliquant code pour (i) une ARN polymérase dépendant de l'ARN qui peut transcrire l'ARN à partir de la molécule d'ARN auto-répliquant et (ii) un immunogène.

10. Liposome selon la revendication 9, dans lequel la molécule d'ARN auto-répliquant comporte deux cadres de lecture ouverts, le premier codant pour une réplicase d'alphavirus et le second codant pour l'immunogène.

11. Liposome selon l'une quelconque des revendications précédentes, dans lequel la molécule d'ARN a une longueur de 9 000 à 12 000 nucléotides.

12. Liposome selon l'une quelconque des revendications précédentes, dans lequel l'immunogène peut susciter une réponse immunitaire *in vivo* à l'encontre d'une bactérie, d'un virus, d'un champignon ou d'un parasite.

13. Composition pharmaceutique comprenant un liposome selon l'une quelconque des revendications précédentes.

14. Liposome selon l'une quelconque des revendications 1 à 12 ou composition pharmaceutique selon la revendication 13, destiné(e) à être utilisé(e) pour provoquer une réponse immunitaire protectrice chez un vertébré.
